# EUROPEAN PATENT SPECIFICATION

(11) **EP 2 294 069 B1**
(45) Date of publication and mention of the grant of the patent: **02.09.2015**
(21) Application number: 09743667.9
(22) Date of filing: 07.05.2009
(51) Int. Cl.: C07D 471/04, C07D 471/06, C07D 471/16, A61P 27/00, A61P 9/00, A61K 31/4745

(54) **THERAPEUTICALLY USEFUL SUBSTITUTED HYDROPYRIDO[3,2,1-IJ]QUINOLINE COMPOUNDS**
THERAPEUTISCH NÜTZLICHE SUBSTITUIERTE HYDROPYRIDO[3,2,1-IJ]CHINOLINVERBINDUNGEN
COMPOSÉS HYDROPYRIDO[3,2,1-IJ]QUINOLÉINE SUBSTITUÉS THÉRAPEUTIQUEMENT UTILES

(30) Priority: 08.05.2008 US 51533 P; 01.05.2009 US 433978
(43) Date of publication of application: 16.03.2011
(73) Proprietor: ALLERGAN, INC., Irvine, CA 92612 (US); Sinha, Santosh C., Ladera Ranch, CA 92694 (US); Bhat, Smita, Irvine, CA 92614 (US); Chow, Ken, Newport Coast, CA 92657 (US); Beard, Richard L., Newport Beach, CA 92660 (US); Donello, John E., Dana Point, CA 92629 (US); Garst, Michael E., Newport Beach, CA 92660 (US)
(72) Inventor: SINHA, Santosh, C., Ladera Ranch, CA 92694 (US); BHAT, Smita, Irvine, CA 92614 (US); CHOW, Ken, Newport Coast, CA 92657 (US); BEARD, Richard, L., Newport Beach, CA 92660 (US); DONELLO, John, E., Dana Point, CA 92629 (US); GARST, Michael, E., Newport Beach, CA 92660 (US)
(74) Representative: Hoffmann Eitle
(86) International application number: PCT/US2009/043149
(87) International publication number: WO 2009/137681

(56) References cited:
- WO-A-03/062392
- WO-A-2004/026864
- WO-A-2004/037213
- WO-A-2007/056155
- LEE S ET AL: "Synthesis and anti-angiogenesis activity of coumarin derivatives" BIOORGANIC & MEDICINAL CHEMISTRY LETTERS, PERGAMON, ELSEVIER SCIENCE, GB, vol. 16, no. 17, 1 September 2006 (2006-09-01), pages 4596-4599, XP025107342 ISSN: 0960-894X [retrieved on 2006-09-01]
- HOLT JASON J ET AL: "A microwave-assisted synthesis of julolidine-9-carboxamide derivatives and their conversion to chalcogenoxanthones via directed metalation." THE JOURNAL OF ORGANIC CHEMISTRY 30 MAR 2007, vol. 72, no. 7, 30 March 2007 (2007-03-30), pages 2690-2693, XP002538089 ISSN: 0022-3263
- DATABASE CHEMCATS [Online] CHEMICAL ABSTRACTS SERVICE, COLUMBUS, US; RN 773874-23-0 1 January 2009 (2009-01-01), XP002538090 retrieved from STN
- DATABASE CHEMCATS [Online] CHEMICAL ABSTRACTS SERVICE, COLUMBUS, US; RN 311324-17-1 24 March 2009 (2009-03-24), XP002538091 retrieved from STN
- DATABASE CHEMCATS [Online] CHEMICAL ABSTRACTS SERVICE, COLUMBUS, US; RN 773135-85-6 1 January 2009 (2009-01-01), XP002538092 retrieved from STN
- DATABASE CHEMCATS [Online] CHEMICAL ABSTRACTS SERVICE, COLUMBUS, US; RN 773129-68-3 1 January 2009 (2009-01-01), XP002538093 retrieved from STN
- DATABASE CHEMCATS [Online] CHEMICAL ABSTRACTS SERVICE, COLUMBUS, US; RN 773869-60-6 1 January 2009 (2009-01-01), XP002538094 retrieved from STN

## Description

### Field of the Invention

The present invention provides novel substituted hydropyrido[3,2,1-ij]quinoline compounds, which are useful for the treatment of mammals with diseases and conditions that are alleviated by sphingosine-1-phosphate (S1P) receptor modulation.

### Background of the Invention

Sphingosine is a compound having the chemical structure shown in the general formula described below, in which Y¹ is hydrogen. It is known that various sphingolipids, having sphingosine as a constituent, are widely distributed in the living body including on the surface of cell membranes of cells in the nervous system.

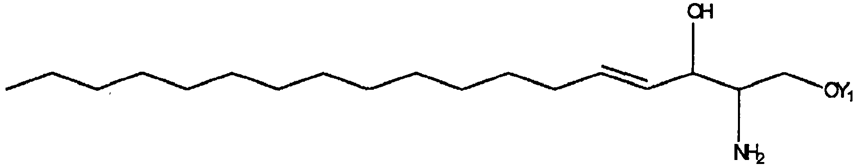

A sphingolipid is one of the lipids having important roles in the living body. A disease called lipidosis is caused by accumulation of a specified sphingolipid in the body. Sphingolipids present on cell membranes function to regulate cell growth; participate in the development and differentiation of cells; function in nerves; are involved in the infection and malignancy of cells; etc. Many of the physiological roles of sphingolipids remain to be solved. Recently the possibility that ceramide, a derivative of sphingosine, has an important role in the mechanism of cell signal transduction has been indicated, and studies about its effect on apoptosis and cell cycle have been reported.

Sphingosine-1-phosphate is an important cellular metabolite, derived from ceramide that is synthesized de novo or as part of the sphingomeyeline cycle (in animals cells). It has also been found in insects, yeasts and plants.

The enzyme, ceramidase, acts upon ceramides to release sphingosine, which is phosphorylated by sphingosine kinase, a ubiquitous enzyme in the cytosol and endoplasmic reticulum, to form sphingosine-1-phosphate. The reverse reaction can occur also by the action of sphingosine phosphatases, and the enzymes act in concert to control the cellular concentrations of the metabolite, which concentrations are always low. In plasma, such concentration can reach 0.2 to 0.9 µM, and the metabolite is found in association with the lipoproteins, especially the HDL. It should also be noted that sphingosine-1-phosphate formation is an essential step in the catabolism of sphingoid bases.

Like its precursors, sphingosine-1-phosphate is a potent messenger molecule that perhaps uniquely operates both intra- and inter-cellularly, but with very different functions from ceramides and sphingosine. The balance between these various sphingolipid metabolites may be important for health. For example, within the cell, sphingosine-1-phosphate promotes cellular division (mitosis) as opposed to cell death (apoptosis), which it inhibits. Intracellularly, it also functions to regulate calcium mobilization and cell growth in response to a variety of extracellular stimuli. Current opinion appears to suggest that the balance between sphingosine-1-phosphate and ceramide and/or sphingosine levels in cells is critical for their viability.

In common with the lysophospholipids, especially lysophosphatidic acid, with which it has some structural similarities, sphingosine-1-phosphate exerts many of its extra-cellular effects through interaction with five specific G protein-coupled receptors on cell surfaces, known as endothelium differentiation gene receptors ("Edg" or "S1 P" receptors).

S1P3 receptor is one of the receptors interacting with sphingosine-1-phosphate. S1P3 receptor, alone or together with other S1P receptors, involves in many critical biological processes, such as the growth of new blood vessels, vascular maturation, cardiac development and immunity, as well as for directed cell movement. S1P3 receptor modulators are needed for therapeutic uses.

Methods of treating conditions associated with an endothelial gene differentiation (Edg) receptor are disclosed in WO 03/062392 A2. Preferred Edg-3 receptor modulators disclosed in this document are 1,2,3,5,6,7-hexahydropyrido[3,2,1-ij]quinoline derivative.

### Summary of the Invention

An aspect of the present invention is a compound represented by the following formula or a pharmaceutically-acceptable salt or tautomer thereof: 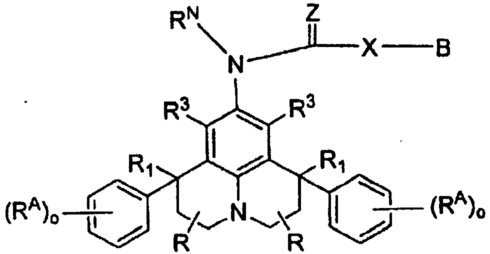 wherein:
o is 0, 1, 2 or 3;
X is O, S or NR^{N};
ZisO;
B is C₁₋₆ alkyl, C₁₋₆ haloalkyl or C₁₋₆ alkenyl;
each R, R¹ and R³ is independently H or C₁₋₆ alkyl;
each R^{A} is independently H, C₁₋₁₂ alkyl, C₁₋₁₂ alkenyl, C₁₋₁₂ alkynyl, halo, C₁-₁₂ halohydrocarbyl, C₁₋₁₂ hydroxyalkyl, C₃₋₁₂ cyclic hydrocarbyl, or heteroaryl;
and
R^{N} is H or C₁₋₆ alkyl.

Specific compounds represented by the formula above are defined in the claims.

Another aspect of the invention is a compound selected from the following compounds, or a pharmaceutically-acceptable salt or tautomer thereof:

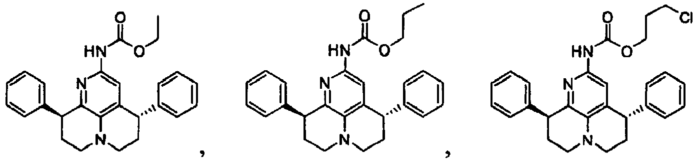

The compounds of the invention have been found to modulate sphingosine-1-phosphate (S1P) receptor activity, in particularly inhibit S1P3 receptor. These compounds are useful for the treatment of mammals, including human beings, with a range of conditions and diseases that are alleviated by S1P modulation, such as ocular diseases and conditions (glaucoma, elevated intraocular pressure, dry eye, and optical neurodegenerative diseases), cardiovascular diseases and conditions, pulmonary diseases and conditions, skin conditions, angiogenesis, inflammation, sepsis and pain.

### Detailed Description of the Invention

The compounds of the invention are useful for the treatment of mammals, including humans, with a range of conditions and diseases that are alleviated by S1 P modulation, including glaucoma, elevated intraocular pressure, ischemic neuropathies, optic neuropathy, visceral pain, corneal pain, headache pain, migraine, cancer pain, back pain, irritable bowel syndrome pain, muscle pain and pain associated with diabetic neuropathy, diabetic retinopathy, other retinal degenerative conditions, dry eye, angiogenesis and wounds. Other uses include:
ocular applications such as retinopathy of prematurity, diabetic retinopathy, optic neuropathy, glaucomatous retinopathy, macular degeneration, choroidal neovascularization, ocular wound, and retinal edema treatment;
cardiovascular applications such as congestive heart failure, cardiac arrhythmia, atherosclerosis, and bradycardia treatment;
pulmonary applications such as asthma, chronic obstructive pulmonary disease, acute lung injury, acute respiratory distress syndrome, idiopathic pulmonary fibrosis, and ventilation-induced lung injury treatment; and
skin applications such as scar-less wound and cosmetic wound treatment.
For the purposes of this disclosure, "treat," "treating," or "treatment" refer to the diagnosis, cure, mitigation, treatment, or prevention of disease or other undesirable condition.

The compounds of present invention may be identified either by their chemical structures and/or chemical names. If the chemical structure and the chemical name conflict, the chemical structure is determinative of the identity of the compound.

The compounds of the invention may contain one or more chiral centers and/or double bonds and therefore, may exist as stereoisomers, such as double-bond isomers, enantiomers or diastereomers. Accordingly, the chemical structures depicted herein encompass all possible enantiomers and stereoisomers, including the stereoisomerically pure form and enantiomeric and stereoisomeric mixtures. The compounds of the invention may also exist in several tautomeric forms, including but not limiting to, the enol form, the keto form and mixtures thereof. Accordingly, the chemical structures depicted herein encompass all possible tautomeric forms. The compounds of the invention also include isotopically labeled compounds where one or more atoms have an atomic mass different from the atomic mass conventionally found in nature.

Further, the compounds of the invention should be construed broadly to include their pharmaceutically acceptable salts, non-covalent complexes, and combinations thereof, unless otherwise indicated.

A pharmaceutically acceptable salt is any salt of the parent compound that is suitable for administration to a mammal, including humans. A pharmaceutically acceptable salt also refers to any salt which may form *in vivo* as a result of administration of an acid, another salt, or a prodrug which is converted into an acid or salt. A salt comprises one or more ionic forms of the compound, such as a conjugate acid or base, associated with one or more corresponding counter-ions. Salts can form from or incorporate one or more deprotonated acidic groups (e.g. carboxylic acids), one or more protonated basic groups (e.g. amines), or both (e.g. zwitterions).

The compounds may exist in different solid forms than those that may result from practicing the procedures described herein. For example, different solid forms may be polymorphs, different kinds of amorphous solid forms, glasses, and the like.

Non-covalent complexes are complexes that may form between the compound and one or more additional chemical species that do not involve a covalent bonding interaction between the compound and the additional chemical species. They may or may not have a specific ratio between the compound and the additional chemical species. Examples include solvates, hydrates and charge transfer complexes.

Hydrocarbyl consists of carbon and hydrogen, wherein each carbon has 4 covalent bonds and each hydrogen has a single bond to a carbon atom. "Hydrocarbyl fragments" has the same meaning as "hydrocarbyl," but is merely used for convenience for counting purposes. For example, one or more hydrocarbyl fragments means, 1, 2, or more distinct parts that each consists of hydrocarbyl, which may be interrupted by another moiety. For example, a functional group may be attached to 2 distinct hydrocarbyl fragments.

Hydrocarbyl includes alkyl, alkenyl, alkynyl, aryl containing only hydrogen and carbon, and combinations thereof. Hydrocarbyl may be linear, branched, cyclic (aromatic or non-aromatic), or combinations thereof.

Alkyl is a hydrocarbyl having no double bonds. Examples include methyl, ethyl, propyl isomers, butyl isomers, pentyl isomers, hexyl isomers, cyclopropyl, cyclobutyl, cyclopentyl and cyclohexyl.

Alkenyl is a hydrocarbyl having one or more double bonds. Examples include ethenyl, propenyl, butenyl isomers, pentenyl isomers, hexenyl isomers, cyclopentenyl and cyclohexenyl.

Alkynyl is a hydrocarbyl having one or more triple bonds. Examples include ethynyl, propynyl, butynyl isomers, pentynyl isomers, hexynyl isomers, cyclopentynyl and cyclohexynyl.

Aryl is an aromatic ring or ring system. It can be hydrocarbon-aryl or heteroaryl. Examples of hydrocarbon-aryl include phenyl, naphthyl, and biphenyl. Such aryl can be bonded to other moieties within the molecule at any position.

Each hydrogen atom has one covalent bond to carbon (C), nitrogen (N), oxygen (O), or sulfur (S).

Halo or halo atoms are fluorine (F), chlorine (Cl), bromine (Br), and iodine (I). Each halo atom forms a single bond to a carbon atom. Halohydrocarbyl is a hydrocarbyl having one or more F, Cl, Br, or I as substituents.

Heteroaryl is an aromatic ring or ring system containing at least one heteroatom selected from N, O, S, P, and combinations thereof. Examples of heteroaryl include pyridine, pyrazine, pyrimidine, pyridazine, triazine, furan, pyrrole, thiophene, imidazole, pyrazole, oxazole, isoxazole, thiazole, isothiazole, oxadiazole, thiadiazole, naphthalene, quinoline, quinoxaline, quinazoline, cinnoline, isoquinoline, benzofuran, indole, benzothiophene, benzimidazole, indazole, benzoxazole, benzisoxazole, benzothiazole, isobenzofuran, isoindole, tetraline, chromane, isochromane, thiochromane, chromene, isochromene, thiochromene, indane, indene, coumarine and coumarinone. Such heteroaryl group can be bonded to other moieties within the molecule at any position.

The compounds of the present invention can be combined with at least one other therapeutic agent that is already known in the art. The compounds of the invention and the other therapeutic agent(s) can act additively, or more preferably, synergistically.

The invention is further defined by reference to the following examples, which describe the preparation schemes and methods for obtaining specific compounds of the invention, and the assays for testing the biological activities of these compounds. It will be apparent to those skilled in the art that many modifications, both to the preparation schemes and assays, may be practiced.

### EXAMPLES:

### Organic Synthesis:

Reaction Schemes A, B, and C are examples of the preparation methods for obtaining the compounds of the invention.

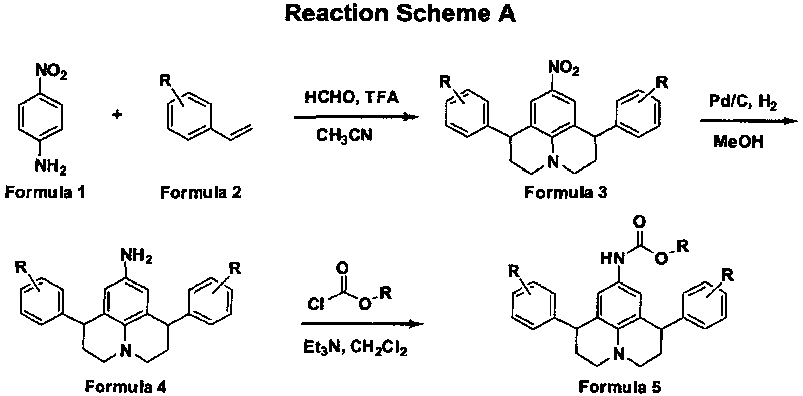

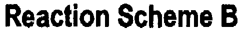

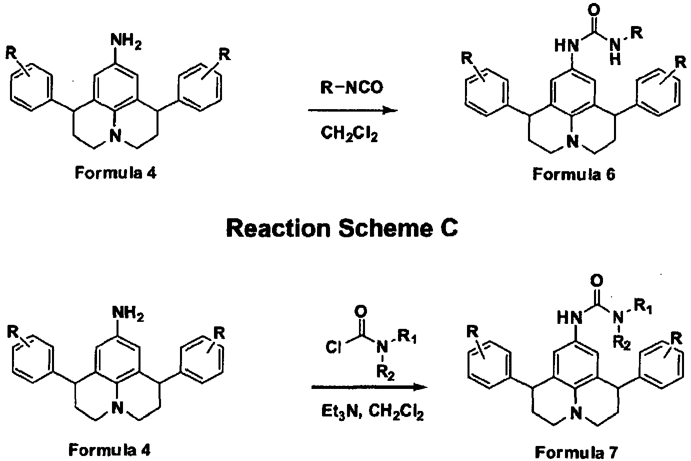

### Example A

### Method A1: Preparation of methyl 1,7-diphenyl-1,2,3,5,6,7-hexahydropyrido[3,2,1-ij]quinolin-9-ylcarbamate (272)

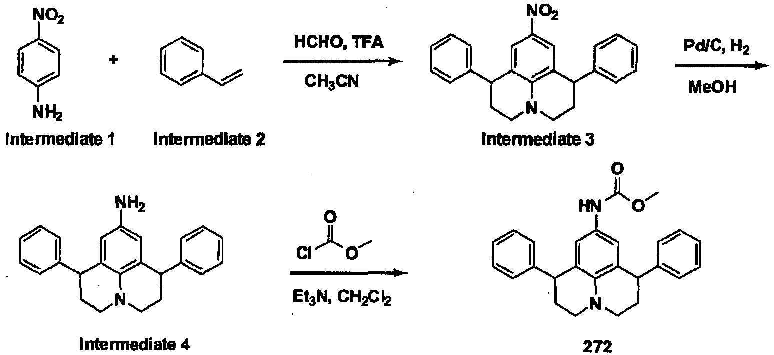

To a solution 4-Nitroaniline (**Intermediate 1**) (1.8 g, 10 mmol) in acetonitrile (8 mL) was added one equivalent of trifluoroacetic acid (1.14 g, 10mmol). To this suspension was added with stirring a heterogeneous mixture of styrene (**Intermediate 2**), (5.74 mL, 50 mmol) and 37% formaldehyde solution (4.06 mL, 50 mmol) under argon, which gave a yellow precipitate. The precipitate failed to redissolve after 30 min. of stirring at room temperature, so the mixture was heated at reflux under argon for further 30 min, during which time the precipitate re-dissolved. The reaction mixture was cooled to room temperature. The precipitate was filtered and wash with acetonitrile gave yellow solid, 9-nitro-1,7-diphenyl-1,2,3,5,6,7-hexahydropyrido[3,2,1-ij]quinoline (**Intermediate 3**), (1.53 g, 41 %).

A solution of 9-nitro-1,7-diphenyl-1,2,3,5,6,7-hexahydropyrido[3,2,1-ij]quinoline (**Intermediate 3**), (1.2 g, 7.06 mmol), in MeOH (100 mL) was subjected to hydrogenation reaction by the action of 10% Pd/C (120 mg) under H₂ balloon at room temperature for 12 h. The mixture was filtered through Celite and freed of solvent under reduced pressure to get 1,7-diphenyl-1,2,3,5,6,7-hexahydropyrido[3,2,1-ij]quinolin-9-amine **(Intermediate 4**) as a solid, (1.08 g, 98%).

To a solution 1,7-diphenyl-1,2,3,5,6,7-hexahydropyrido[3,2,1-ij]quinolin-9-amine (**Intermediate 4**), (207 mg, 0.608 mmol) in dichloromethane (10 mL) was added three equivalent of triethyl amine (0.252 mL, 1.8 mmol), followed by methyl chloroformate (0.071 mL, 0.91 mmol) under argon at 0 °C. The reaction mixture was then stirred at room temperature for overnight. The mixture was quenched with water (30 mL). The residue was isolated in a typical aqueous workup and purified by MPLC (medium pressure liquid chromatography) using silica gel column with 10 to 15 % EtOAc:Hexane to give methyl 1,7-diphenyl-1,2,3,5,6,7-hexahydropyrido[3,2,1-ij]quinolin-9-ylcarbamate (**272**), (181 mg 75%).¹H NMR (300 MHz, CDCl₃) δ ppm 2.00 - 2.18 (m, 2 H) 2.22 - 2.39 (m, 2 H) 3.03 - 3.22 (m, 4 H) 3.50 - 3.64 (m, 3 H) 3.54 - 3.65 (m, 3 H) 4.05 - 4.23 (m, 2 H) 6.61 (br. s., 2 H) 7.08 - 7.38 (m, 10 H).

### Method A2: Preparation of methyl (1S,7S)-1,7-dimethyl-1,7-diphenyl-1,2,3,5,6,7-hexahydropyrido[3,2,1-ij]quinolin-9-ylcarbamate, (829), and methyl (1R7S)-1,7-dimethyl-1,7-diphenyl-1,2,3,5.6,7-hexahydropyrido[3,2,1-ij]quinolin-9-ylcarbamate (353)

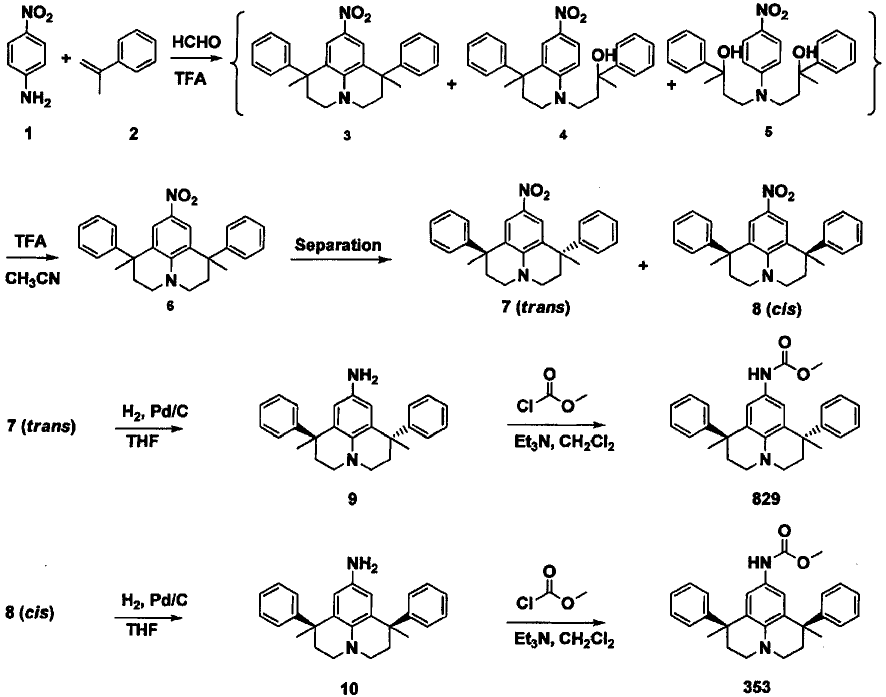

To a solution 4-Nitroaniline (1) (4.1 g, 30 mmol) in acetonitrile (30 mL) was added one equivalent of trifluoroacetic acid (2.3 mL, 30 mmol). To this suspension was added with stirring a heterogeneous mixture of styrene (2), (19.4 mL, 150 mmol) and 37% formaldehyde solution (12.2 mL, 150 mmol) under argon gave yellow precipitate. The precipitate had failed to re-dissolve after 30min. of stirring at room temperature, so the mixture was heated at reflux under argon for further 30min, during which time the precipitate re-dissolved. The reaction mixture was cooled to room temperature. After general workup afforded mixture of three intermediates **3** (836 mg), **4** (2 g), and **5** (4.2 g), confirmed by Mass Spectra and ¹HNMR (see ref. John M. Mellor; et al; Tetrahedron, 1995, 6115). These intermediates were then converted into the cycloadduct product by heating at reflux with trifluoroacetic acid in acetonitrile yielded 1,7-dimethyl-9-nitro-1,7-diphenyl-1,2,3,5,6,7-hexahydropyrido[3,2,1-*ij*]quinoline (**6**) (7.036 g, 59%) as a solid. This solid product (**6**) was then separated to *trans* and *cis* isomers by washing with ether gave *trans* (**7**), (4.0 g) and hexane:CH₂Cl₂ gave *cis* (**8**), (2.8 g).

A mixture of (*1S*,*7S*)-1,7-dimethyl-9-nitro-1,7-diphenyl-1,2,3,5,6,7-hexahydropyrido[3,2,1-*ij*]quinoline (7), (2 g, 5 mmol), in THF (60 mL) was subjected to hydrogenation reaction by the action of 10% Pd/C (200 mg) under H₂ balloon at room temperature for 12 h. The mixture was filtered through Celite and freed of solvent under reduced pressure to get (*1S*,*7S*)-1,7-dimethyl-1,7-diphenyl-1,2,3,5,6,7-hexahydropyrido[3,2,1-*ij*]quinolin-9-amine (**9**) as a solid, (1.5 g, 100%) on the basis of recovered starting material (**7**), (390 mg).

Following a procedure similar to that for (**9**) gained (*1R, 7S*)-1,7-dimethyl-1,7-diphenyl-1,2,3,5,6,7-hexahydropyrido[3,2,1-*ij*]quinolin-9-amine (**10**) as a solid (2.55 g, 100% yield) from (**8**).

To a solution (*1S*,*7S*)-1,7-dimethyl-9-nitro-1,7-diphenyl-1,2,3,5,6,7-hexahydropyrido[3,2,1-*ij*]quinoline (7), (115 mg, 0.31 mmol), in dicloromethane (15 mL) was added three equivalent of triethyl amine (0.129 mL, 0.93 mmol), followed by methyl chloroformate (0.031 mL, 0.406 mmol) under argon at 0 °C. The reaction mixture was then stirred at room temperature for overnight. The mixture was quenched with water (30 mL). The residue was isolated in a typical aqueous workup and purified by MPLC (medium pressure liquid chromatography) using silica gel column with 10 to 15 % EtOAc:Hexane to give methyl (*1S*,*7S*)-1,7-dimethyl-1,7-diphenyl-1,2,3,5,6,7-hexahydropyrido[3,2,1-*ij*]quinolin-9-ylcarbamate (**829**), (69 mg 52%). 1H NMR (300 MHz, Acetone-d6) δ ppm 1.72 (s, 6H) 1.89 - 2.03 (m, 2 H) 2.17 - 2.30 (m, 2 H) 2.73 - 2.88 (m, 2 H) 2.92 - 3.03 (m, 2 H) 3.56 (s, 3 H) 7.05 - 7.33 (m, 12 H) 8.02 (br. s., 1 H).

Following a procedure similar to that for (**829**), gained (*1R*,*7S*)-1,7-dimethyl-1,7-diphenyl-1,2,3,5,6,7-hexahydropyrido[3,2,1-*ij*]quinolin-9-ylcarbamate (**353**), (130g, 62% yield) from (**10**). 1H NMR (300 MHz, Acetone-d6) δ ppm 1.74 (s, 6 H) 1.98 - 2.10 (m, 2 H) 2.13 - 2.24 (m, 2 H) 2.78 - 2.92 (m, 2 H) 2.92 - 3.05 (m, 2 H) 3.55 (s, 3 H) 7.06 (s, 2 H) 7.12 - 7.34 (m, 10 H) 8.02 (br. s., 1 H).

The following compounds were prepared according to the **Reaction Scheme A** and with the steps as shown in **Example A** above.

### Ethyl 1,7-diphenyl-1,2,3,5,6,7-hexahydropyrido[3,2,1-ij]quinolin-9-ylcarbamate, 273

¹H NMR (300 MHz, CDCl₃) δ ppm 1.11 (t, *J*=7.03 Hz, 3 H) 2.02 - 2.17 (m, 2 H) 2.21 - 2.37 (m, 2 H) 3.04 - 3.17 (m, 4 H) 4.04 (q, 2 H) 4.09 - 4.21 (m, 2 H) 6.61 (br. s., 2 H) 7.08-7.39 (m, 10 H)

### Isobutyl 1,7-diphenyl-1,2,3,5,6,7-hexahydropyrido[3,2,1-ij]quinolin-9-ylcarbamate, 274

¹H NMR (300 MHz, CDCl₃) δ ppm 0.94 (d, J=6.74 Hz, 6 H) 1.84 - 2.02 (m, 1 H) 2.02 - 2.17 (m, 2 H) 2.20 - 2.37 (m, 2 H) 3.31-3.22 (m, 4 H) 4.19-4.14 (m, 2 H) 6.63 (br. s., 2 H) 7.11 - 7.24 (m, 5 H) 7.25 - 7.37 (m, 5 H)

### Propyl 1,7-diphenyl-1,2,3,5,6,7-hexahydropyrido[3,2,1-ij]quinolin-9-ylcarbamate, 275

¹H NMR (300 MHz, CDCl₃) δ ppm 0.85 (t, J=6.89 Hz, 3 H) 1.46 - 1.64 (m, 2 H) 2.00 - 2.18 (m, 2 H) 2.21 - 2.37 (m, 2 H) 3.03 - 3.19 (m, 4 H) 3.88 - 4.00 (m, 2 H) 4.09 - 4.23 (m, 2 H) 6.61 (br. s., 2 H) 7.07 - 7.37 (m, 10 H)

### Butyl 1,7-diphenyl-1,2,3,5,6,7-hexahydropyrido[3,2,1-ij]quinolin-9-ylcarbamate, 276

¹H NMR (300 MHz, CDCl₃) δ ppm 0.87 (t, *J*=7.0 Hz, 3 H) 1.30 (br. s., 2 H) 1.42 - 1.56 (m, 2 H) 2.02 - 2.17 (m, 2 H) 2.19 - 2.39 (m, 2 H) 3.03 - 3.17 (m, 4 H) 3.91 - 4.03 (m, 2 H) 4.10 - 4.22 (m, 2 H) 6.61 (br. s., 2 H) 7.08 - 7.38 (m, 10 H)

### Phenyl 1,7-diphenyl-1,2,3,5,6,7-hexahydropyrido[3,2,1-ij]quinolin-9-ylcarbamate, 277

¹H NMR (300 MHz, CDCl₃) δ ppm 2.05 - 2.19 (m, 2 H) 2.21 - 2.40 (m, 2 H) 3.04 - 3.20 (m, 4 H), 4.11 - 4.24 (m, 2 H) 6.71 (br. s., 2 H) 6.99 - 7.44 (m, 15 H)

### Benzyl 1,7-diphenyl-1,2,3,5,6,7-hexahydropyrido[3,2,1-ij]quinolin-9-ylcarbamate, 278

¹H NMR (300 MHz, CDCl₃) δ ppm 1.91 - 2.09 (m, 2 H) 2.10 - 2.29 (m, 2 H) 2.93 - 3.13 (m, 4 H) 4.01 - 4.15 (m, 2 H) 4.92 (s, 2 H) 6.56 (br. s., 2 H) 7.01 - 7.32 (m, 15 H)

### Propyl 1,7-bis(3-fluorophenyl)-1,2,3,5,6,7-hexahydropyrido[3,2,1-ij]quinolin-9-ylcarbamate, 094

1 H NMR (300 MHz, CD₃OD) δ ppm 0.89 (t, 7.0 Hz, H) 1.47 - 1.67 (m, 2 H) 1.99 - 2.14 (m, 2 H) 2.19 - 2.37 (m, 2 H) 3.08 (t, *J*=5.71 Hz, 4 H) 3.91 (t, *J*=6.74 Hz, 2 H) 4.17 (t, *J*=6.01 Hz, 2 H) 6.67 (br. s., 2 H) 6.81 - 7.04 (m, 6 H) 7.22 - 7.38 (m, 2 H)

### Ethyl 1,7-bis(3-fluorophenyl)-1,2,3,5,6,7-hexahydropyrido[3,2,1-ij]quinolin-9-ylcarbamate, 093

1H NMR (300 MHz, CD₃OD) δ ppm 1.15 (t, 7.0 Hz, H) 1.98 - 2.16 (m, 2 H) 2.18 - 2.37 (m, 2 H) 2.96 - 3.18 (m, 4 H) 4.00 (q, *J*=7.13 Hz, 2 H) 4.19 (t, *J*=5.86 Hz, 2 H) 6.68 (br. s., 2 H) 6.77 - 7.01 (m, 6 H) 7.20 - 7.35 (m, 2 H)

### Butyl 1,7-bis(3-fluorophenyl)-1,2,3,5,6,7-hexahydropyrido[3,2,1-ij]quinolin-9-ylcarbamate, 202

1H NMR (300 MHz, Acetone-d₆) δ ppm 0.86 (t, 7.0 Hz, 6 H) 1.20 - 1.39 (m, 2 H) 2.06 - 2.15 (m, 2 H) 2.21 - 2.39 (m, 2 H) 3.02 - 3.17 (m, 4 H) 3.94 (t, *J*=6.59 Hz, 2 H) 4.22 (t, *J*=5.86 Hz, 2 H) 6.83 (br. s., 2 H) 6.88 - 7.09 (m, 6 H) 7.28 - 7.42 (m, 2 H)

### Methyl 1,7-bis(3-fluorophenyl)-1,2,3,5,6,7-hexahydropyrido[3,2,1-ij]quinolin-9-ylcarbamate, 779

1H NMR (300 MHz, CD₃OD) δ ppm 2.01 - 2.15 (m, 2 H) 2.21 - 2.37 (m, 2 H) 3.09 (t, *J*=5.71 Hz, 4 H) 3.57 (s, 3 H) 4.18 (t, *J*=6.15 Hz, 2 H) 6.67 (s, 2 H) 6.81 - 7.03 (m, 6 H) 7.25 - 7.38 (m, 2 H)

### 2-fluoroethyl 1,7-diphenyl-1,2,3,5,6,7-hexahydropyrido[3,2,1-ij]quinolin-9-ylcarbamate, 095

1 H NMR (300 MHz, CD₃OD) δ ppm 1.99 - 2.16 (m, 2 H) 2.19 - 2.36 (m, 2 H) 3.00 - 3.16 (m, 4 H) 4.09 - 4.27 (m, 4 H) 4.29 - 4.36 (m, 1 H) 4.46 - 4.52 (m, 1 H) 6.66 (br. s., 2 H) 7.07 - 7.21 (m, 6 H) 7.21 - 7.33 (m, 4 H)

### But-3-enyl 1,7-diphenyl-1,2,3,5,6,7-hexahydropyrido[3,2,1-ij]quinolin-9-ylcarbamate, 354

1H NMR (300 MHz, Acetone-d₆) δ ppm 2.04 - 2.15 (m, 2 H) 2.18 - 2.41 (m, 4 H) 3.02 - 3.18 (m, 4 H) 3.89 - 4.01 (m, 2 H) 4.90 - 5.14 (m, 2 H) 5.69 - 5.88 (m, 1 H) 6.78 (br. s., 2 H) 7.08 - 7.25 (m, 6 H) 7.23 - 7.38 (m, 4 H)

### But-2-ynyl 1,7-diphenyl-1,2,3,5,6,7-hexahydropyrido[3,2,1-ij]quinolin-9-ylcarbamate, 353

1H NMR (300 MHz, Acetone-d₆) δ ppm 1.74 (s, 3 H) 2.06 - 2.13 (m, 2 H) 2.19 - 2.34 (m, 2 H) 3.02 - 3.20 (m, 4 H) 4.19 (t, *J*=6.01 Hz, 2 H) 4.52 (q, *J*=2.54 Hz, 2 H) 6.78 (s, 2 H) 7.12 - 7.24 (m, 6 H) 7.26 - 7.35 (m, 4 H)

### Prop-2-ynyl 1,7-diphenyl-1,2,3,5,6,7-hexahydropyrido[3,2,1-ij]quinolin-8-ylcarbamate, 352

1 H NMR (300 MHz, Acetone-d₆) δ ppm 2.05 - 2.15 (m, 2 H) 2.20 - 2.36 (m, 2 H) 2.90 (t, *J*=2.49 Hz, 1 H) 3.00 - 3.20 (m, 4 H) 4.19 (t, *J*=6.01 Hz, 2 H) 4.59 (d, *J*=2.34 Hz, 2 H) 6.78 (s, 2 H) 7.11 - 7.24 (m, 6 H) 7.25 - 7.37 (m, 4 H)

### 4-chlorobutyl 1,7-diphenyl-1,2,3,5,6,7-hexahydropyrido[3,2,1-ij]quinolin-8-ylcarbamate, 206

1H NMR (300 MHz, Acetone-d₆) δ ppm 1.66 -1.96 (m, 4 H) 2.06 - 2.11 (m, 2 H) 2.22 - 2.32 (m, 2 H) 3.04 - 3.17 (m, 4 H) 3.58 (t, *J*=6 Hz, 2H) 3.96 (t, *J*=6.3 Hz, 2H) 4.19 (t, *J*=6.3 Hz, 2 H) 6.77 (s, 2 H) 7.16 - 7.20 (m, 6 H) 7.22 - 7.33 (m, 4 H)

### 3-chloropropyl 1,7-diphenyl-1,2,3,5,6,7-hexahydropyrido[3,2,1-ij]quinolin-9-ylcarbamate, 205

1 H NMR (300 MHz, CD₃OD) δ ppm 1.91 - 2.14 (m, 4 H) 2.20 - 2.34 (m, 2 H) 3.02 - 3.15 (m, 4 H) 4.07 (t, *J*=6.01 Hz, 2 H) 4.11 - 4.21 (m, 2 H) 6.64 (br. s., 2 H) 7.09 - 7.21 (m, 6 H) 7.23 - 7.31 (m, 4 H)

### Ethyl (1S,7S)-1,7-dimethyl-1,7-diphenyl-1,2,3,5,6,7-hexahydropyrido[3,2,1-ij]quinolin-9-ylcarbamate, 941

1 H NMR (300 MHz, Acetone-d6) δ ppm 1.16 (t, *J*=7.18 Hz, 3 H) 1.74 (s, 6 H) 1.90 - 2.01 (m, 2 H) 2.16 - 2.32 (m, 2 H) 2.73 - 2.89 (m, 2 H) 2.90 - 3.03 (m, 2 H) 4.02 (q, *J*=7.13 Hz, 2 H) 7.06 - 7.32 (m, 12 H) 8.02 (br. s., 1 H)

### Propyl (1S,7S)-1,7-dimethyl-1,7-diphenyl-1,2,3,5,6,7-hexahydropyrido[3,2,1-ij]quinolin-9-ylcarbamate, 942

1 H NMR (300 MHz, Acetone-d6) δ ppm 0.88 (t, *J*=7.33 Hz, 3 H) 1.46 - 1.65 (m, 2 H) 1.72 (s, 6 H) 1.88 - 2.03 (m, 2 H) 2.18 - 2.32 (m, 2 H) 2.70 - 2.88 (m, 2 H) 2.89 - 3.03 (m, 2 H) 3.94 (t, *J*=6.59 Hz, 2 H) 7.06 - 7.33 (m, 12 H) 8.02 (br. s., 1 H)

### Butyl (1S,7S)-1,7-dimethyl-1,7-diphenyl-1,2,3,5,6,7-hexahydropyrido[3,2,1-ij]quinolin-9-ylcarbamate, 943

1H NMR (300 MHz, Acetone-d6) δ ppm 0.88 (t, *J*=7.33 Hz, 3 H) 1.27 - 1.42 (m, 1 H) 1.45 - 1.61 (m, 1 H) 1.67 - 1.77 (s, 6 H) 1.88 - 2.03 (m, 2 H) 2.18 - 2.31 (m, 2 H) 2.72 - 2.89 (m, 4 H) 2.90 - 3.02 (m, 2 H) 3.93 - 4.05 (m, 2 H) 7.06 - 7.32 (m, 12 H) 8.01 (br. s., 1 H)

### 2-fluoroethyl (1S,7S)-1,7-dimethyl-1,7-diphenyl-1,2,3,5,6,7-hexahydropyrido[3,2,1-ij]quinolin-9-ylcarbamate, 944

1H NMR (300 MHz, Acetone-d6) δ ppm 1.73 (s, 6 H) 1.91 - 2.02 (m, 2 H) 2.19 - 2.32 (m, 2 H) 2.73 - 2.85 (m, 2 H) 2.88 - 3.04 (m, 2 H) 4.16 - 4.26 (m, 1 H) 4.25 - 4.36 (m, 1 H) 4.44 - 4.55 (m, 1 H) 4.60 - 4.68 (m, 1 H) 7.09 - 7.31 (m, 12 H) 8.20 (br. s., 1 H)

### 3-chloropropyl (1S,7S)-1,7-dimethyl-1,7-diphenyl-1,2,3,5,6,7-hexahydropyrido[3,2,1-ijjquinolin-9-ylcarbamate, 945

1H NMR (300 MHz, Acetone-d6) δ ppm 1.73 (s, 6 H) 1.89 - 2.03 (m, 2 H) 2.18 - 2.33 (m, 2 H) 2.18 - 2.31 (m, 2 H) 2.75 - 2.88 (m, 2 H) 2.90 - 3.03 (m, 2 H) 3.58 - 3.70 (m, 2 H) 4.08 - 4.19 (m, 2 H) 7.03 - 7.32 (m, 12 H) 8.11 (br. s., 1 H)

### Ethyl (1R,7S)-1,7-dimethyl-1,7-diphenyl-1,2,3,5,6,7-hexahydropyrido[3,2,1-ij]quinolin-9-ylcarbamate, 354

1H NMR (300 MHz, Acetone-d6) δ ppm 1.15 (t, *J*=7.03 Hz, 3 H) 1.74 (s, 6 H) 1.97 - 2.11 (m, 2 H) 2.11 - 2.24 (m, 2 H) 2.73 - 2.91 (m, 2 H) 2.92 - 3.03 (m, 2 H) 3.94 - 4.08 (m, 2 H) 7.08 (br. s., 2 H) 7.12 - 7.35 (m, 10 H) 7.98 (br. s., 1 H)

### Propyl (1R,7S)-1,7-dimethyl-1,7-diphenyl-1,2,3,5,6,7-hexahydropyrido[3,2,1-ij]quinolin-8-ylcarbamate, 355

1H NMR (300 MHz, Acetone-d6) δ ppm 0.87 (t, *J*=7.33 Hz, 3 H) 1.46 - 1.62 (m, 2 H) 1.74 (s, 6 H) 1.98 - 2.09 (m, 2 H) 2.10 - 2.23 (m, 2 H) 2.82 - 2.91 (m, 2 H) 2.91 - 3.06 (m, 2 H) 3.87 - 3.98 (m, 2 H) 7.08 (s, 2 H) 7.14 - 7.35 (m, 10 H) 8.01 (br. s., 1 H)

### Butyl (1R,7S)-1,7-dimethyl-1,7-diphenyl-1,2,3,5,6,7-hexahydropyrido[3,2,1-ij]quinolin-9-ylcarbamate, 356

1H NMR (300 MHz, Acetone-d6) δ ppm 0.88 (t, *J*=7.33 Hz, 3 H) 1.24 - 1.41 (m, 2 H) 1.47 - 1.60 (m, 2 H) 1.74 (s, 6 H) 1.97 - 2.11 (m, 2 H) 2.08 - 2.24 (m, 2 H) 2.78 - 2.90 (m, 2 H) 2.89 - 3.04 (m, 2 H) 3.91 - 4.01 (m, 2 H) 7.09 (br. s., 2 H) 7.13 - 7.34 (m, 10 H) 8.00 (br. s., 1 H)

### 3-chloropropyl (1R,7S)-1,7-dimethyl-1,7-diphenyl-1,2,3,5,6,7-hexahydropyrido[3,2,1-ij]quinolin-8-ylcarbamate, 357

1H NMR (300 MHz, Acetone-d6) δ ppm 1.74 (s, 6 H) 1.97 - 2.09 (m, 2 H) 2.11 - 2.25 (m, 2 H) 2.79 - 2.93 (m, 4 H) 2.91 - 3.04 (m, 2 H) 3.56 - 3.72 (m, 2 H) 4.05 - 4.18 (m, 2 H) 7.07 (br. s., 2 H) 7.10 - 7.34 (m, 10 H) 8.08 (s, 1 H)

### But-3-enyl (1R,7S)-1,7-dimethyl-1,7-diphenyl-1,2,3,5,6,7-hexahydropyrido[3,2,1-ij]quinolin-8-ylcarbamate, 358

1H NMR (300 MHz, Acetone-d6) δ ppm 1.74 (s, 6 H) 1.93 - 2.12 (m, 2 H) 2.11 - 2.23 (m, 2 H) 2.23 - 2.37 (m, 2 H) 2.81 - 2.90 (m, 2 H) 2.91 - 3.06 (m, 2 H) 4.02 (t, *J*=6.74 Hz, 2 H) 4.91 - 5.14 (m, 2 H) 5.68 - 5.87 (m, 1 H) 7.09 (br. s., 2 H) 7.12 - 7.35 (m, 10 H) 8.03 (br. s., 1 H)

### methyl (1S,7S)-1,7-bis(4-fluorophenyl)-1,7-dimethyl-1,2,3,5,6,7-hexahydropyrido[3,2,1-ij]quinolin-8-ylcarbamate, 674

¹H NMR (300 MHz, Acetone-d6): δ = 1.71 (s, 6H) 1.92 - 2.07 (m, 2H) 2.19 - 2.27 (m, 2H) 2.76 - 2.84 (m, 2H) 2.95 - 3.02 (m, 2H) 3.57 (s, 3H) 6.98 - 7.22 (m, 10H)

### butyl (1S,7S)-1,7-bis(4-fluorophenyl)-1,7-dimethyl-1,2,3,5,6,7-hexahydropyrido[3,2,1-ij]quinolin-9-ylcarbamate, 672

¹H NMR (300 MHz, Acetone-d6): δ = 0.88 (t, *J*=7.5 Hz, 3 H) 1.51 - 1.56 (m, 2H) 1.33 - 1.38 (m, 2H) 1.73 (s, 6H) 1.92 - 2.06 (m, 2H) 2.21 - 2.27 (m, 2H) 2.81 - 2.84 (m, 2H) 2.94 - 3.27 (m, 2H) 3.97 - 4.43 (m, 2 H) 6.98 - 7.22 (m, 10H)

### 5-methyl (1S,7S)-1,7-dimethyl-1,7-diphenyl-1,2,3,5,6,7-hexahydropyrido[3,2,1-ij]quinolin-9-ylcarbamothioate, 583

¹H NMR (300 MHz, Acetone-d6): δ = 1.68 (s, 6H) 2.02 (s, 3H) 2.20 - 1.88 (m, 4H) 2.95 - 3.01 (m, 2H) 2.80 - 2.95 (m, 2H) 6.76 (s, 2H) 7.33 - 7.17 (m, 10H)

### 5-propyl (1S,7S)-1,7-dimethyl-1,7-diphenyl-1,2,3,5,6,7-hexahydropyrido[3,2,1-ij]quinolin-9-ylcarbamothioate, 585

¹H NMR (300 MHz, Acetone-d6): δ = 0.91 (t, *J=7.2* Hz, 3 H), 0.97-1.1 (m, 2H) 1.49 - 1.51 (m, 4H) 1.74 (s, 6H) 1.92 - 1.96 (m, 2H) 2.18 - 2.23 (m, 2H) 2.73 - 2.84 (m, 4H) 2.95 - 3.05 (m, 2H) 7.05 (s, 2H) 7.15 - 7.36 (m, 8H)

### 5-methyl (1R,7S)-1,7-dimethyl-1,7-diphenyl-1,2,3,5,6,7-hexahydropyrido[3,2,1-ij]quinolin-9-ylcarbamothioate, 582

¹H NMR (300 MHz, Acetone-d6): δ = 1.73 (s, 6H) 2.20 (s, 3H) 1.98 - 2.07 (m, 2H) 2.14 - 2.17 (m, 2H) 2.80 - 2.88(m, 2H) 2.95 - 3.01 (m, 2H) 7.13 - 7.32 (m, 12H)

### S-propyl (1R,7S)-1,7-dimethyl-1,7-diphenyl-1,2,3,5,6,7-hexahydropyrido[3,2,1-ij]quinolin-9-ylcarbamothioate, 584

¹H NMR (300 MHz, Acetone-d6): δ = 1.40 (t, *J* =9Hz, 3H) 2.05 - 2.12 (m, 2H) 2.28 (s, 6H) 2.66 - 2.72 (m, 6H) 2.51 - 2.64 (m, 2H) 3.47 - 3.54 (m, 2H) 3.31 - 3.39 (m, 2H) 7.59-7.80 (m, 12H)

### S-methyl (1S,7S)-1,7-bis(4-fluorophenyl)-1,7-dimethyl-1,2,3,5,6,7-hexahydropyrido[3,2,1-ij]quinolin-9-ylcarbamothioate, 671

¹H NMR (300 MHz, Acetone-d6): δ = 1.73 (s, 6H) 2.19 - 2.21 (m, 2H) 2.24-2.27 (m, 2H) 2.81 (s, 3H) 2.76 - 2.86 (m, 2H) 2.96 - 3.03 (m,2H) 6.93 - 7.22 (m, 10H)

### S-propyl (1S,7S)-1,7-bis(4-fluorophenyl)-1,7-dimethyl-1,2,3,5,6,7-hexahydropyrido[3,2,1-ij]quinolin-9-ylcarbamothioate, 673

¹H NMR (300 MHz, Acetone-d6): δ = 0.92 (t, *J*=9 Hz, 3 H) 1.08 - 2.11 (m, 2 H) 1.50 - 1.60 (m, 4 H) 1.70 (s, 6 H) 1.90 -1.98 (m, 1 H) 2.17 - 2.25 (m, 3 H) 2.73-2.78 (m, 1 H) 2.93 - 3.00 (m,1 H) 6.96 - 7.19 (m, 10 H)

### Example B

### Method B1: Preparation of 1-(1,7-diphenyl-1,2,3,5,6,7-hexahydropyrido[3,2,1-ij]quinolin-9-yl)-3-ethylurea (484)

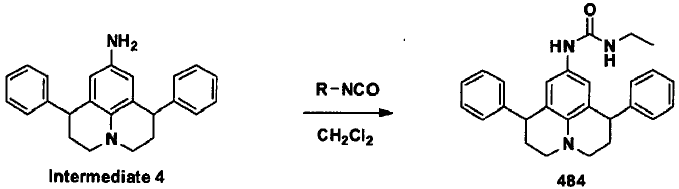

To a solution 1,7-diphenyl-1,2,3,5,6,7-hexahydropyrido[3,2,1-ij]quinolin-9-amine (**Intermediate 4**), (102 mg, 0.30 mmol) in dichloromethane (15 mL) was added ethyl isocyanate (0.026 mL, 0.33 mmol), mmol) under argon at 0 °C. The reaction mixture was then stirred at room temperature for overnight. The solvent was removed under reduced pressure and purified by MPLC (medium pressure liquid chromatography) using silica gel column with 15 to 20 % EtOAc:Hexane to get 1-(1,7-diphenyl-1,2,3,5,6,7-hexahydropyrido[3,2,1-ij]quinolin-9-yl)-3-ethylurea (**484**) (120 mg 97%). ¹H NMR (300 MHz, CDCl₃) δ ppm 0.91 (t, *J*=7.18 Hz, 3 H) 2.05 - 2.20 (m, 2 H) 2.21 - 2.39 (m, 2 H) 2.96 - 3.28 (m, 6 H) 4.07 - 4.19 (m, 2 H) 4.35 (br. s., 1 H) 5.58 (s, 1 H) 6.43 (s, 2 H) 7.06 - 7.36 (m, 10 H)

### Method B2: Preparation of 1-((1S,7S)-1,7-dimethyl-1,7-diphenyl-1,2,3,5,6,7-hexahydropyrido[3,2,1-ij]quinolin-9-yl)-3-ethylurea (249)

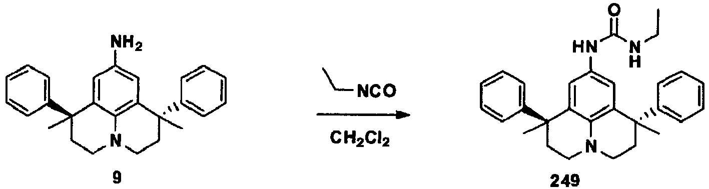

To a solution (*1*S,*7S*)-1,7-dimethyl-9-amino-1,7-diphenyl-1,2,3,5,6,7-hexahydropyrido[3,2,1-*ij*]quinoline (**9**), (110 mg, 0.30 mmol) in dicloromethane (15 mL) was added ethyl isocyanate (0.026 mL, 0.328 mmol), mmol) under argon at 0 °C. The reaction mixture was then stirred at room temperature for overnight. The solvent was removed under reduced pressure and purified by MPLC) using silica gel column with 15 to 20 % EtOAc:Hexane to get 1-((*1S*,*7S*)-1,7-dimethyl-1,7-diphenyl-1,2,3,5,6,7-hexahydropyrido[3,2,1-*ij*]quinolin-9-yl)-3-ethylurea (**249**), (96 mg 73%). 1 H NMR (300 MHz, Acetone-d6) δ ppm 1.00 (t, J=7.18 Hz, 3 H) 1.71 (s, 6H) 1.91 - 2.02 (m, 2 H) 2.15 - 2.31 (m, 2 H) 2.75 - 2.87 (m, 2 H) 2.90 - 3.03 (m, 2 H) 3.03 - 3.16 (m, 2 H) 6.98 (s, 2H) 7.09 - 7.31 (m, 10 H)

The following compounds were prepared according to the **Reaction Scheme B** and with the steps as shown in **Example B** above.

### 1-Butyl-3-(1,7-diphenyl-1,2,3,5,6,7-hexahydropyrido[3,2,1-ij]quinolin-9-yl)urea, 485

¹H NMR (300 MHz, CDCl₃) δ ppm 0.85 (t, *J*=7 Hz, 3 H) 1.04 - 1.38 (m, 4 H) 2.05 - 2.19 (m, 2 H) 2.19 - 2.38 (m, 2 H) 2.89 - 3.29 (m, 6 H) 4.14 (t, *J*=6.15 Hz, 2 H) 4.38 (br. s., 1 H) 5.61 (s, 1 H) 6.43 (s, 2 H) 7.05 - 7.38 (m, 10 H)

### 1-(1,7-diphenyl-1,2,3,5,6,7-hexahydropyrido[3,2,1-ij]quinolin-9-yl)-3-pentylurea, 486

¹H NMR (300 MHz, CDCl₃) δ ppm 0.84 (t, *J*=7.1₈ Hz, 3 H) 1.03 - 1.33 (m, 6 H) 2.04 - 2.19 (m, 2 H) 2.20 - 2.38 (m, 2 H) 2.90 - 3.28 (m, 6 H) 4.07 - 4.20 (m, 2 H) 4.38 (br. s., 1 H) 5.58 (s, 1 H) 6.43 (s, 5 H) 7.07 - 7.39 (m, 10 H)

### 1-(1,7-diphenyl-1,2,3,5,6,7-hexahydropyrido[3,2,1-ij]quinolin-9-yl)-3-hexylurea, 487

¹H NMR (300 MHz, CDCl₃) δ ppm 0.86 (t, *J*=7.1 Hz3 H) 1.06 - 1.33 (m, 8 H) 2.00 - 2.20 (m, 2 H) 2.21 - 2.40 (m, 2 H) 2.93 - 3.29 (m, 6 H) 4.02 - 4.17 (m, 2 H) 4.29 - 4.45 (m, 1 H) 5.57 (br. s., 1 H) 6.43 (br. s., 2 H) 7.03 - 7.40 (m, 10 H)

### 1-(2-chloroethyl)-3-(1,7-diphenyl-1,2,3,5,6,7-hexahydropyrido[3,2,1-ij]quinolin-9-yl)urea, 769

¹H NMR (300 MHz, CD₃OD) δ ppm 1.99 - 2.15 (m, 2 H) 2.19 - 2.35 (m, 2 H) 3.04 - 3.15 (m, 4 H), 3.49 (m, 2 H) 3.41 - 3.50 (m, 2 H) 4.11 - 4.22 (m, 2 H) 6.55 (s, 2 H) 7.09 - 7.21 (m, 5 H) 7.21 - 7.32 (m, 5 H)

### 1-allyl-3-(1,7-diphenyl-1,2,3,5,6,7-hexahydropyrido[3,2,1-ij]quinolin-9-yl)urea, 773

¹H NMR (300 MHz, CD₃OD) δ ppm 2.00 - 2.14 (m, 2 H) 2.20 - 2.36 (m, 2 H) 3.04 - 3.16 (m, 4 H) 3.58 - 3.69 (m, 2 H) 4.11 - 4.23 (m, 2 H) 4.94 - 5.13 (m, 1 H) 5.73 (m, 1 H) 6.56 (s, 2 H) 7.09 - 7.21 (m, 5 H) 7.23 - 7.31 (m, 5 H)

### 1-tert-butyl-3-(1,7-diphenyl-1,2,3,5,6,7-hexahydropyrido[3,2,1-ij]quinolin-9-yl)urea, 148

¹H NMR (300 MHz, CDCl₃) δ ppm 7.12 - 7.33 (m, 10H) 6.41 (s, 2H) 4.14 (t, *J*=6 Hz, 2H) 3.14 - 3.19 (m, 4H) 2.25 - 2.30 (m, 2H) 2.10 - 2.17 (m, 2H) 2.25 - 2.33 (m, 2H)

### 1-(1,7-diphenyl-1,2,3,5,6,7-hexahydropyrido[3,2,1-ij]quinolin-9-yl)-3-(furan-3-ylmethyl)urea, 258

¹H NMR (300 MHz, CD₃OD) δ ppm 7.12 - 7.33 (m, 11H) 6.56 (s, 2H), 6.25 - 6.27 (m, 1H) 6.08 - 6.10 (m, 1H) 4.19 (s, 2H) 4.16 (t, *J*=6 Hz, 2H) 3.07 - 3.12 (m, 4H) 2.01 - 2.11 (m, 2H) 2.27 - 2.29 (m, 2H)

### 1-((1S,7S)-1,7-dimethyl-1,7-diphenyl-1,2,3,5,6,7-hexahydropyrido[3,2,1-ij]quinolin-9-yl)-3-propylurea, 250

¹H NMR (300 MHz, Acetone-d6) δ ppm 0.83 (t, *J*=6.8 Hz, 3 H) 1.33 - 1.47 (m, 2 H) 1.72 (s, 6H) 1.88 - 2.02 (m, 2 H) 2.17 - 2.30 (m, 2 H) 2.77 - 2.88 (m, 2 H) 2.90 - 3.10 (m, 4 H) 6.98 (s, 2 H) 7.10 - 7.30 (m, 10 H)

### 1-((1S,7S)-1,7-dimethyl-1,7-diphenyl-1,2,3,5,6,7-hexahydropyrido[3,2,1-ij]quinolin-9-yl)-3-butylurea, 251

¹H NMR (300 MHz, Acetone-d6) δ ppm 0.86 (t, J=6.8 Hz, 3 H) 1.20 - 1.45 (m, 4 H) 1.70 (s, 6H) 1.90 - 2.02 (m, 2 H) 2.15 - 2.30 (m, 2 H) 2.79 - 2.88 (m, 2 H) 2.90 - 3.02 (m, 2 H) 3.02 - 3.15 (m, 2 H) 6.98 (s, 2 H) 7.09 - 7.33 (m, 10 H)

### 1-allyl-3-((1S,7S)-1,7-dimethyl-1,7-diphenyl-1,2,3,5,6,7-hexahydropyrido[3,2,1-ij]quinolin-9-yl)urea, 252

¹H NMR (300 MHz, Acetone-d6) δ ppm 1.73 (s, 6 H) 1.87 - 2.02 (m, 2 H) 2.16 - 2.30 (m, 2 H) 2.74 - 2.87 (m, 2 H) 2.89 - 3.01 (m, 2 H) 3.67 - 3.78 (m, 2 H) 4.91 - 5.16 (m, 2 H) 5.73 - 5.90 (m, 1 H) 7.00 (s, 2 H) 7.06 - 7.35 (m, 10 H)

### 1-t-butyl-3-((1S,7S)-1,7-dimethyl-1,7-diphenyl-1,2,3,5,6,7-hexahydropyrido[3,2,1-i]quinolin-9-yl)urea, 581

¹H NMR (300 MHz, Acetone-d6): δ = 0.71 (s, 9H) 1.20 (s, 6H) 1.97-2.02 (m, 2H) 2.07 - 2.16 (m, 2H) 2.74 - 2.80 (m, 2H) 2.93 - 2.97 (m, 2H) 6.79 (s, 2H) 7.36 - 7.17 (m, 10H)

### 1-((1R,7S)-1,7-dimethyl-1,7-diphenyl-1,2,3,5,6,7-hexahydropyrido[3,2,1-ij]quinolin-9-yl)-3-ethylurea, 463

¹H NMR (300 MHz, Acetone-d6) δ ppm 1.00 (t, *J*=7.18 Hz, 3 H) 1.73 (s, 6 H) 1.94 - 2.03 (m, 2 H) 2.10 - 2.25 (m, 2 H) 2.79 - 2.90 (m, 2 H) 2.92 - 3.04 (m, 2 H) 3.10 (t, *J*=6.45 Hz, 2 H) 5.29 (br. s., 1 H) 6.93 (s, 2 H) 7.07 - 7.34 (m, 10 H)

### 1-((1R,7S)-1,7-dimethyl-1,7-diphenyl-1,2,3,5,6,7-hexahydropyrido[3,2,1-ij]quinolin-9-yl)-3-propylurea, 464

¹H NMR (300 MHz, Acetone-d6) δ ppm 0.82 (t, J=7.00 Hz, 3 H) 1.31 - 1.48 (m, 2 H) 1.75 (s, 6 H) 1.95 - 2.03 (m, 2 H) 2.10 - 2.24 (m, 2 H) 2.75 - 2.89 (m, 4 H) 2.92 - 3.07 (m, 2 H) 5.33 - 5.35 (m, 1 H) 6.95 (s, 2 H) 7.07 - 7.35 (m, 10 H)

### 1-((1R,7S)-1,7dimethyl-1,7-diphenyl-1,2,3,5,6,7-hexahydropyrido[3,2,1-ij]quinolin-9-yl)-3-butylurea, 465

¹H NMR (300 MHz, Acetone-d6) δ ppm 0.86 (t, J=7.18 Hz, 3 H) 1.19 - 1.44 (m, 4 H) 1.73 (s, 6 H) 1.94 - 2.05 (m, 2 H) 2.11 - 2.23 (m, 2 H) 2.77 - 2.92 (m, 4 H) 2.92 - 3.02 (m, 2 H) 3.02 - 3.12 (m, 2 H) 5.31 (br. s., 1 H) 7.09 - 7.34 (m, 10 H)

### 1-t-butyl-3-((1R,7S)-1,7-dimethyl-1,7-diphenyl-1,2,3,5,6,7-hexahydropyrido[3,2,1-ij]quinolin-9-yl)urea, 580

¹H NMR (300 MHz, Acetone-d6): δ = 1.26 (s, 9H) 1.71 (s, 6H) 1.57 - 1.71 (m, 2H) 1.43 - 1.47 (m, 2H) 2.44 - 2.49 (m, 2H) 2.24 - 2.30 (m, 2H) 6.30 (s, 2H) 6.64 - 6.84 (m, 10H)

### Example C

### Method C: Preparation of 3-(1,7-diphenyl-1,2,3,5,6,7-hexahydropyrido[3,2,1-ij]quinolin-9-yl)-1,1-dimethylurea (405)

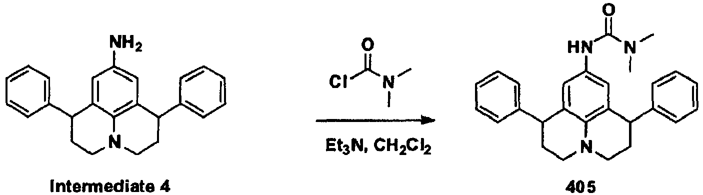

To a solution 1,7-diphenyl-1,2,3;5,6,7-hexahydropyrido[3,2,1-ij]quinolin-9-amine (Intermediate 4), (106 mg, 0.31 mmol) in dichloromethane (10 mL) was added triethyl amine (0.130 mL, 0.933 mmol) followed by dimethylcarbamic chloride (0.043 mL, 0.46 mmol), under argon at 0 °C. The reaction mixture was then stirred at room temperature for overnight. The mixture was quenched with water (30 mL). The residue was isolated in a typical aqueous workup and purified by MPLC (medium pressure liquid chromatography) using silica gel column with 10 to 15 % EtOAc:Hexane to give 3-(1,7-diphenyl-1,2,3,5,6,7-hexahydropyrido[3,2,1-ij]quinolin-9-yl)-1,1-dimethylurea, (**405**), (63 mg 49%). ¹H NMR (300 MHz, CDCl₃) □ ppm 2.01 - 2.16 (m, 2 H) 2.20 - 2.37 (m, 2 H) 2.85 (s, 6 H) 3.00 - 3.15 (m, 4 H) 4.12 - 4.25 (m, 2 H) 5.78 (s, 1 H) 6.62 (s, 2 H) 7.10 - 7.22 (m, 5 H) 7.23 - 7.34 (m, 5 H)

The following compound was prepared according to the **Reaction Scheme C** and with the steps as shown in **Example C** above.

### N-(1,7-diphenyl-1,2,3,5,6,7-hexahydropyrido[3,2,1-ij]quinolin-9-yl)morpholine-4-carboxamide, 983

¹H NMR (300 MHz, CDCl₃) δ ppm 2.03 - 2.17 (m, 2 H) 2.20 - 2.38 (m, 2 H) 2.92 - 3.42 (m, 8 H) 3.53 - 3.65 (m, 4 H) 3.98 - 4.20 (m, 2 H) 6.52 - 6.72 (m, 2 H) 7.08 - 7.23 (m, 5 H) 7.24 - 7.37 (m, 5 H)

### Example D

### Method D: Procedure for the methyl (4R,10R)-4,10-diphenyl-4,5,6,8,9,10-hexahydropyrido[3,2,1-de][1,5]naphthyridin-2 ylcarbamate (179)

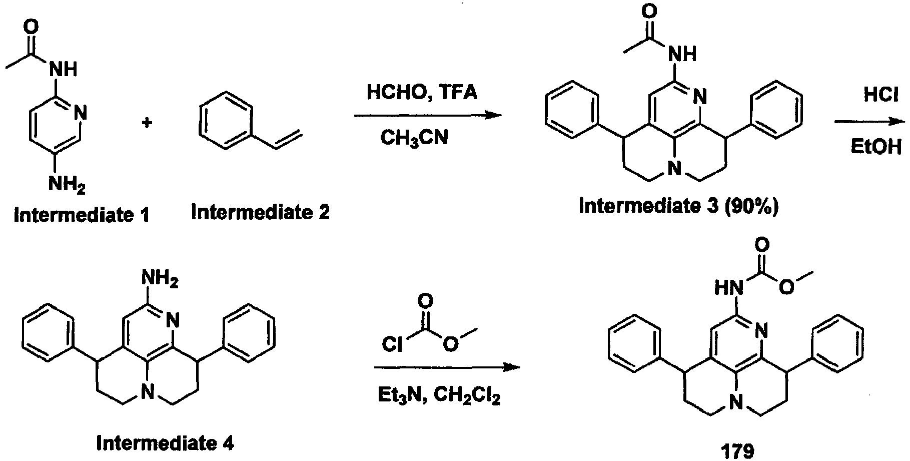

To a solution of N-(5-aminopyridin-2-yl)acetamide (**Intermediate 1**) (2.4 g, 15.8 mmol) in acetonitrile (12 mL) was added one equivalent of trifluoroacetic acid (1.2 mL, 15.8 mmol). To this suspension was added with stirring a heterogeneous mixture of styrene (**Intermediate 2**), (7.2 mL, 63.2 mmol) and 37% formaldehyde solution (5.2 mL, 63.2 mmol) under argon, which gave a yellow precipitate. The precipitate failed to redissolve after 30 min. of stirring at room temperature, so the mixture was heated at reflux under argon for further 30 min, during which time the precipitate redissolved. The reaction mixture was cooled to room temperature. The precipitate was filtered and wash with acetonitrile gave yellow solid, *n*-((4R,10R)-4,10-diphenyl-4,5,6,8,9,10-hexahydropyrido[3,2,1-de][1,5]naphthyridin-2-yl)acetamide, (**Intermediate 3**), (2.78 g).

A mixture of *n*-((4R,10R)-4,10-diphenyl-4,5,6,8,9,10-hexahydropyrido[3,2,1-de][1,5]naphthyridin-2-yl)acetamide, (**Intermediate 3**), (0.550 g, 1.43 mmol), in EtOH (12 mL) was Conc. HcL (1.2 mL). The mixture was strirred at 90°C for two hrs. The mixture was concentrated, neutralized wit aq. NaOH and extracted in CH₂Cl₂, dried (MgSO₄), filtered and concentrated gave (4R,10R)-4,10-diphenyl-4,5,6,8,9,10-hexahydropyrido[3,2,1-de][1,5]naphthyridin-2-amine (**Intermediate 4**) as a solid, (0.380 g).

To a solution (4R,10R)-4,10-diphenyl-4,5,6,8,9,10-hexahydropyrido[3,2,1-de][1,5]naphthyridin-2-amine (**Intermediate 4**), (110 mg, 0.322 mmol) in dichloromethane (10 mL) was added two equivalent of triethyl amine (0.090 mL, 0.644 mmol), followed by methyl chloroformate (0.037 mL, 0.483 mmol) under argon at 0 °C. The reaction mixture was then stirred at room temperature for overnight. The mixture was quenched with water (30 mL). The residue was isolated in a typical aqueous workup and purified by MPLC (medium pressure liquid chromatography) using silica gel column with 10 to 15 % EtOAc:Hexane to give methyl (4R,10R)-4,10-diphenyl-4,5,6,8,9,10-hexahydropyrido[3,2,1-de][1,5]naphthyridin-2-ylcarbamate, (**179**), (20 mg).¹H NMR (300 MHz, CDCl₃) δ ppm 2.00 - 2.18 (m, 2 H) 2.22 - 2.39 (m, 2 H) 3.03 - 3.22 (m, 4 H) 3.50 - 3.64 (m, 3 H) 3.54 - 3.65 (m, 3 H) 4.05 - 4.23 (m, 2 H) 6.61 (br. s., 2 H) 7.08 - 7.38 (m, 10 H)

### Biological Data:

The compounds of the invention are assessed for their ability to activate or block activation of the human S1P3 receptor in T24 cells stably expressing the human S1P3 receptor using the method described in paragraph [0067] of United States Patent Application Publication No. 20070232682, which published on Oct. 4,2007.

Ten thousands cells/well are plated into 384-well poly-D-lysine coated plates one day prior to use. The growth media for the S1P3 receptor expressing cell line is McCoy's 5A medium supplemented with 10% charcoal-treated fetal bovine serum (FBS), 1% antibiotic-antimycotic and 400 µg/ml geneticin. On the day of the experiment, the cells are washed twice with Hank's Balanced Salt Solution supplemented with 20 mM HEPES (HBSS/Hepes buffer). The cells are then dye loaded with 2 µM Fluo-4 diluted in the HBSS/Hepes buffer with 1.25 mM Probenecid and incubated at 37 °C for 40 minutes. Extracellular dye is removed by washing the cell plates four times prior to placing the plates in the FLIPR (Fluorometric Imaging Plate Reader, Molecular Devices). Ligands are diluted in HBSS/Hepes buffer and prepared in 384-well microplates. The positive control, Sphingosine-1-phosphate (S1 P), is diluted in HBSS/Hepes buffer with 4 mg/ml fatty acid free bovine serum albumin. The FLIPR transfers 12.5 µl from the ligand microplate to the cell plate and takes fluorescent measurements for 75 seconds, taking readings every second, and then for 2.5 minutes, taking readings every 10 seconds. Compounds are tested over the concentration range of 0.61 nM to 10,000 nM. Data for calcium ion (Ca⁺²) responses are obtained in arbitrary fluorescence units and not translated into Ca⁺² concentrations. IC₅₀ values (nM) are determined through a linear regression analysis using the Levenburg Marquardt algorithm. Table 1 lists the test results for some of the compounds of the present invention:

**Table 1: Biological Data: Activity Potency of Compounds against Human S1P3 Receptor nM, (IC₅₀), %Inhibition**

| Comp. no. | Structure | S1P3 IC₅₀ | S1P3 % Inhibition |
|---|---|---|---|
| 272 | 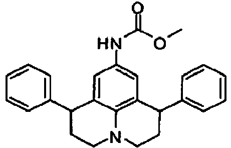 | 8.3 | 102 |
| 273 | 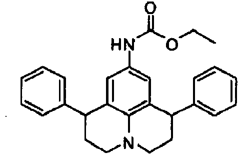 | 63 | 101 |
| 274 | 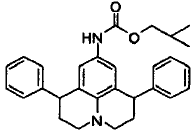 | 64 | 100 |
| 275 | 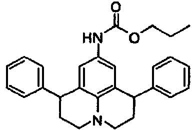 | 12.2 | 100 |
| 276 | 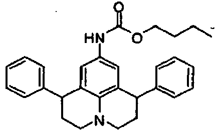 | 8.7 | 101 |
| 484 | 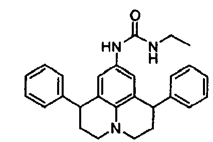 | 77 | 101 |
| 485 | 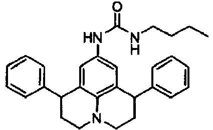 | 52 | 100 |
| 486 | 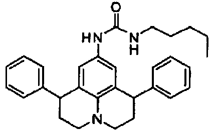 | 82 | 100 |
| 487 | 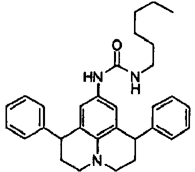 | 301 | 100 |
| 405 | 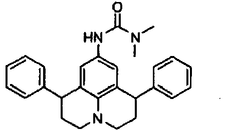 | 131 | 98 |
| 769 | 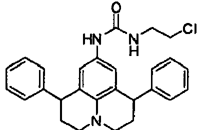 | 374 | 100 |
| 773 | 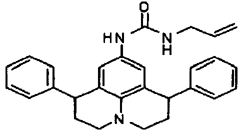 | 160 | 100 |
| 094 | 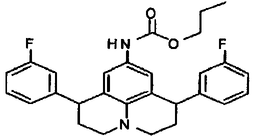 | 234 | 101 |
| 093 | 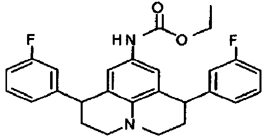 | 48 | 102 |
| (+)-enantiomer | 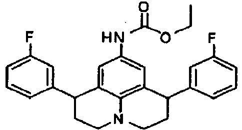 | 3 | 97 |
| (-)-enantiomer | 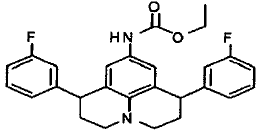 | 230 | 97 |
| 202 | 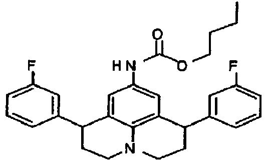 | 272 | 98 |
| 779 | 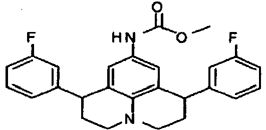 | 23 | 101 |
| 268 | 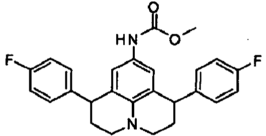 | 5 | 98 |
| (+)-enantiomer | 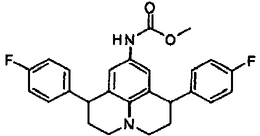 | 1.6 | 98 |
| (-)-enantiomer | 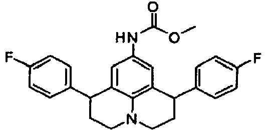 | NA | |
| 095 | 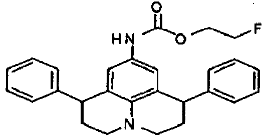 | 56 | 102 |
| 067 | 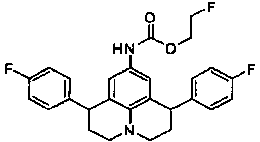 | 8 | 98 |
| (+)-enantiomer | 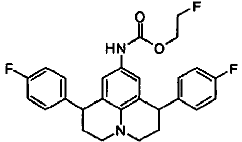 | 3 | 99 |
| (-)-enantiomer | 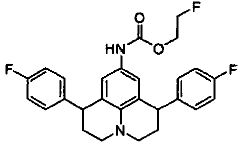 | NA | |
| 40354 | 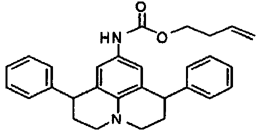 | 16 | 97 |
| 206 | 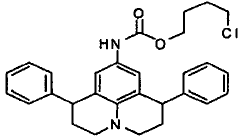 | 62 | 100 |
| 205 | 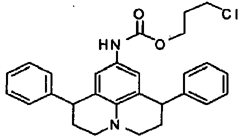 | 8 | 99 |
| (+)-enantiomer | 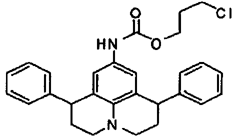 | 4 | 99 |
| (-)-enantiomer | 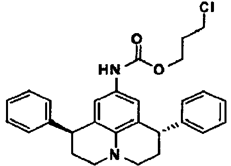 | 1659 | |
| 699 | 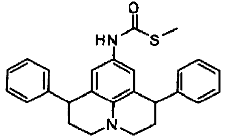 | 4 | 100 |
| (+)-enantiomer | 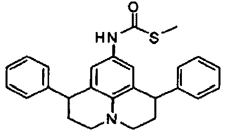 | 1.6 | 98 |
| (-)-enantiomer | 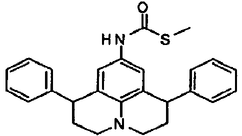 | NA | |
| 700 | 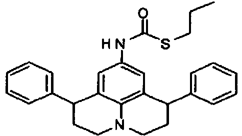 | 7 | 100 |
| (+)-enantiomer | 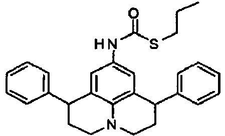 | 13 | 98 |
| (-)-enantiomer | 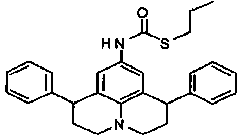 | NA | |
| 148 | 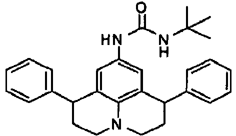 | 38 | 101 |
| 829 | 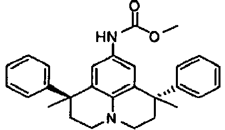 | 7 | 97 |
| 941 | 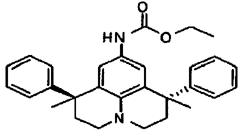 | 13 | 100 |
| 942 | 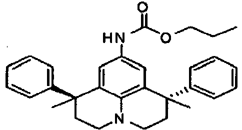 | 72 | 100 |
| 943 | 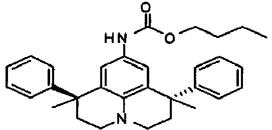 | 75 | 100 |
| 944 | 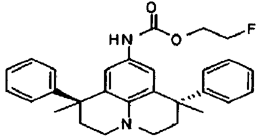 | 27 | 100 |
| 945 | 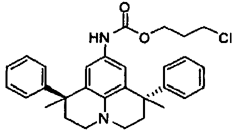 | 68 | 100 |
| 354 | 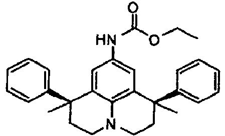 | 36 | 98 |
| 355 | 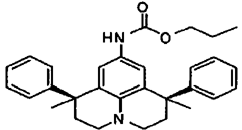 | 316 | 98 |
| 674 | 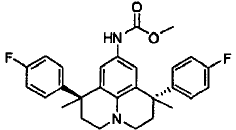 | 46 | 96 |
| 672 | 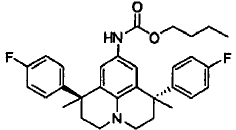 | 86 | 89 |
| 583 | 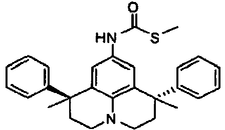 | 10 | 98 |
| 585 | 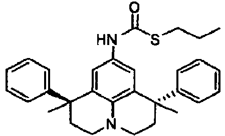 | 5 | 98 |
| 582 | 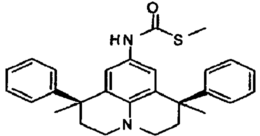 | 4 | 99 |
| 584 | 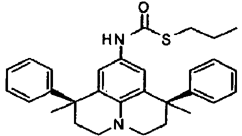 | 16 | 98 |
| 671 | 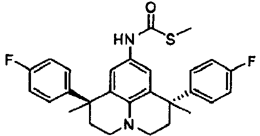 | 25 | 97 |
| 673 | 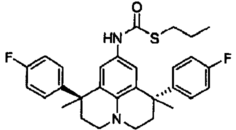 | 62 | 97 |
| 249 | 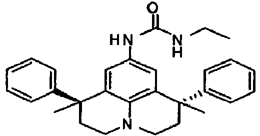 | 194 | 100 |
| 250 | 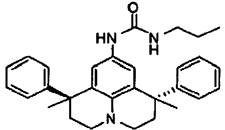 | 523 | 100 |
| 581 | 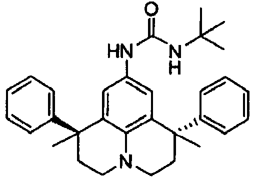 | 349 | 98 |
| 580 | 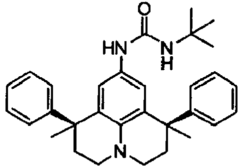 | 62 | 99 |

**TABLE 2: Additional Compounds of the Present Invention**

| **Compound** | **Structure** |
|---|---|
| 3 | 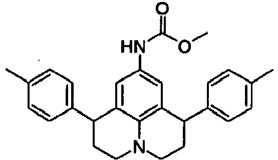 |
| 4 | 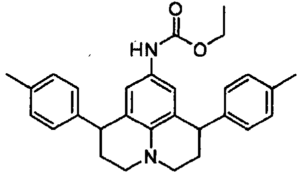 |
| 5 | 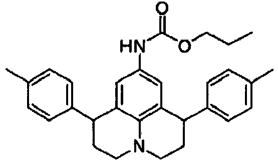 |
| 6 | 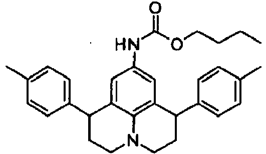 |
| 8 | 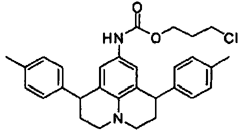 |
| 9 | 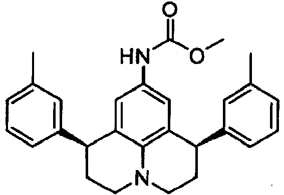 |
| 10 | 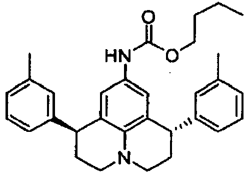 |
| 11 | 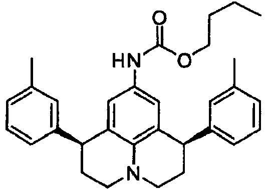 |
| 12 | 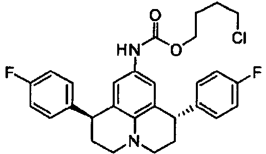 |
| 13 | 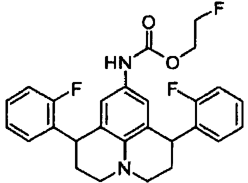 |
| 14 | 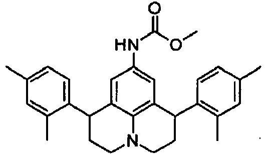 |
| 15 | 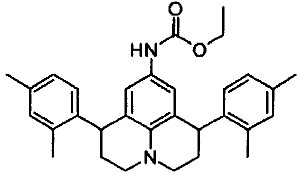 |
| 16 | 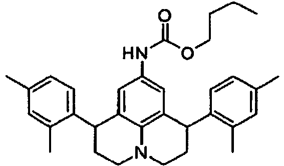 |
| 17 | 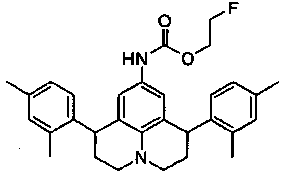 |
| 18 | 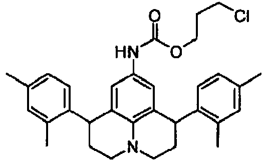 |
| 19 | 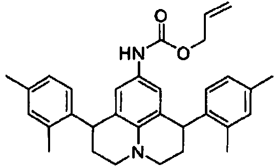 |
| 20 | 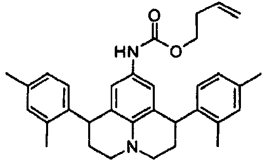 |
| 22 | 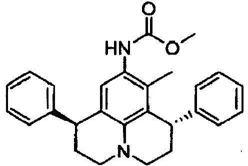 |
| 23 | 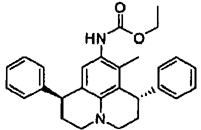 |
| 24 | 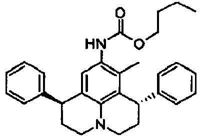 |
| 25 | 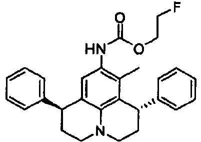 |
| 26 | 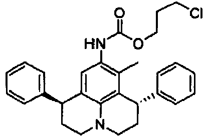 |
| 27 | 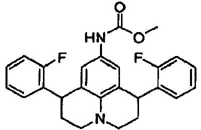 |
| 29 | 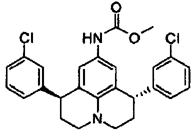 |
| 30 | 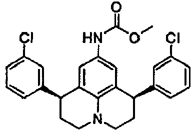 |
| 31 | 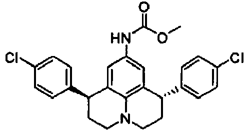 |
| 32 | 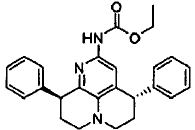 |
| 33 | 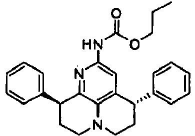 |
| 34 | 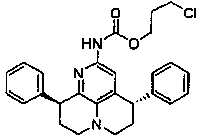 |
| 35 | 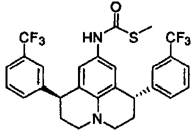 |
| 36 | 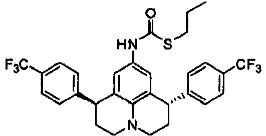 |
| 37 | 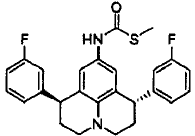 |
| 38 | 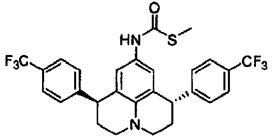 |
| 39 | 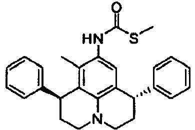 |
| 40 | 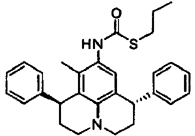 |
| 41 | 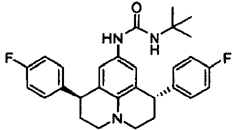 |
| 42 | 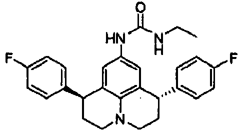 |
| 43 | 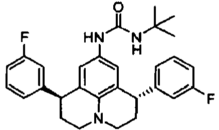 |
| 44 | 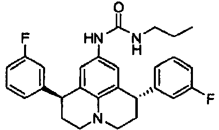 |
| 45 | 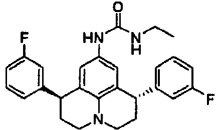 |
| 46 | 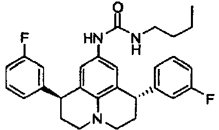 |
| 47 | 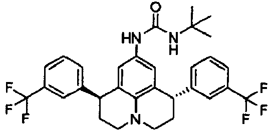 |
| 48 | 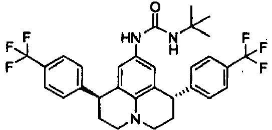 |
| 49 | 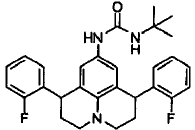 |
| 50 | 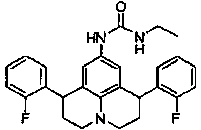 |
| 51 | 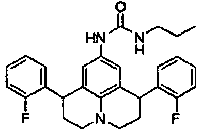 |
| 52 | 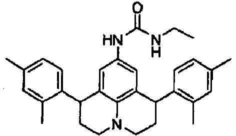 |
| 53 | 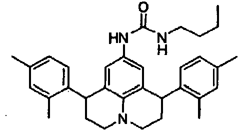 |
| 54 (+) Single enantiomer | 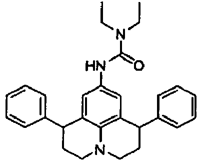 |
| 55 (-) Single enantiomer | 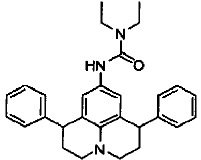 |
| 56 | 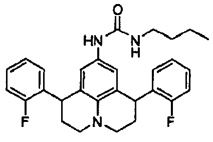 |
| 62 | 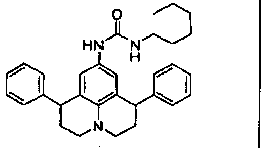 |
| 63 | 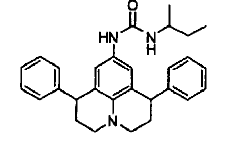 |
| 64 | 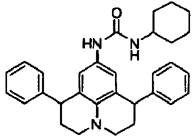 |
| 65 | 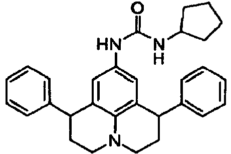 |
| 66 | 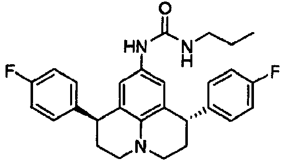 |
| 67 | 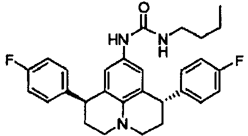 |
| 68 | 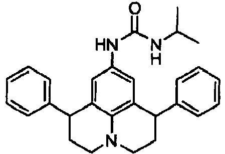 |
| 69 | 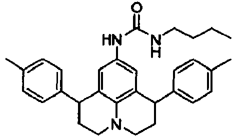 |
| 72 | 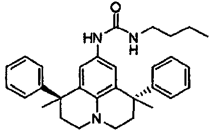 |
| 73 | 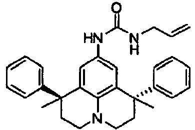 |
| 74 | 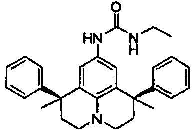 |
| 75 | 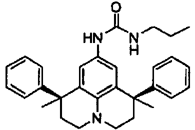 |
| 76 | 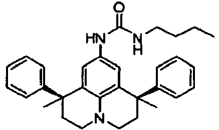 |

## Claims

1. A compound represented by the following formula or a pharmaceutically-acceptable salt or tautomer thereof: 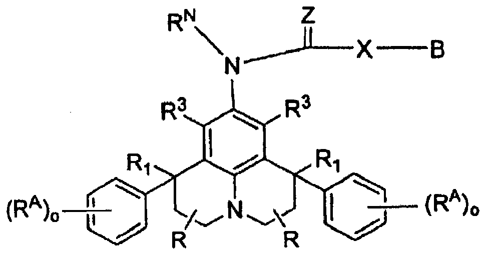 wherein:
o is 0, 1, 2 or 3;
X is O, S or NR^{N};
ZisO;
B is C₁₋₆ alkyl, C₁₋₆ haloalkyl or C₁₋₆ alkenyl;
each R, R¹ and R³ is independently H or C₁₋₆ alkyl;
each R^{A} is independently H, C₁₋₁₂ alkyl, C₁₋₁₂ alkenyl, C₁₋₁₂ alkynyl, halo, C₁₋₁₂ halohydrocarbyl, C₁₋₁₂ hydroxyalkyl, C₃₋₁₂ cyclic hydrocarbyl, or heteroaryl; and
R^{N} is H or C₁₋₆ alkyl.

2. A compound or a salt or tautomer thereof according to Claim 1, which is one of the following compounds or a pharmaceutically-acceptable salt thereof:

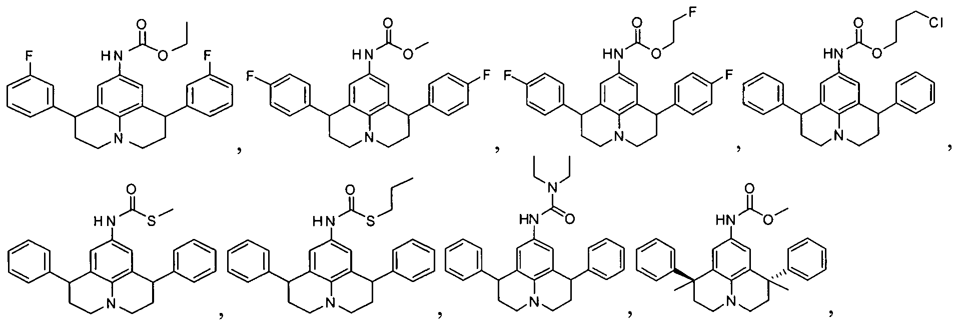

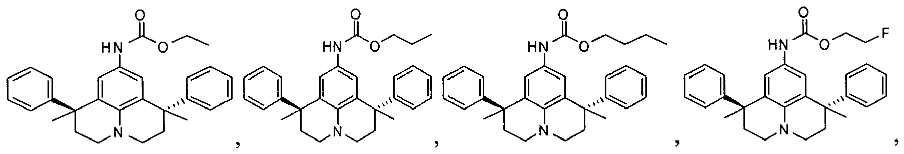

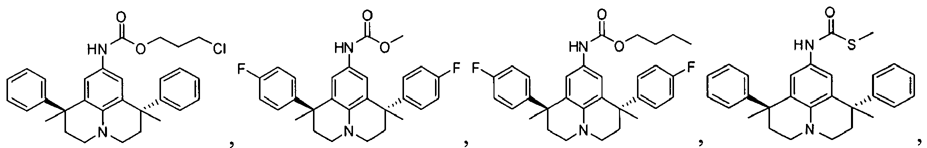

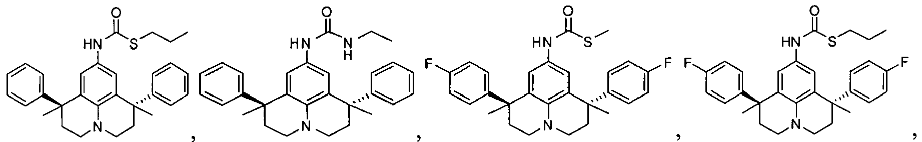

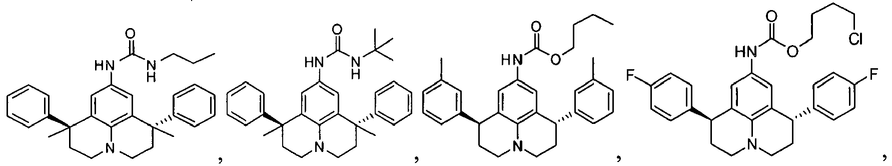

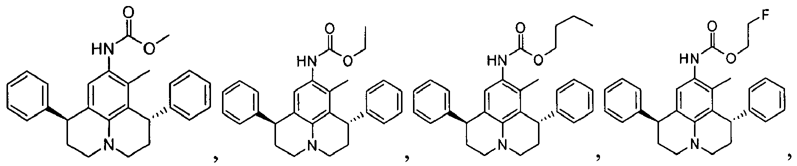

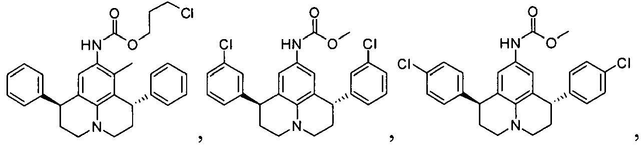

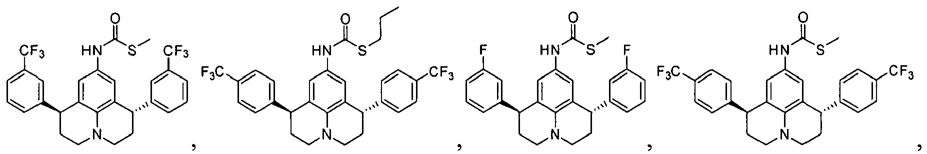

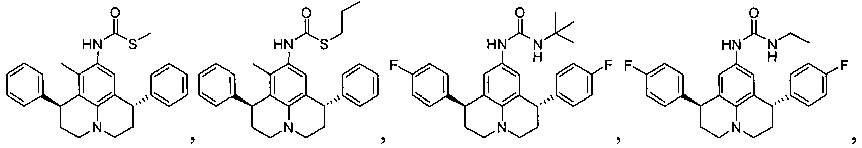

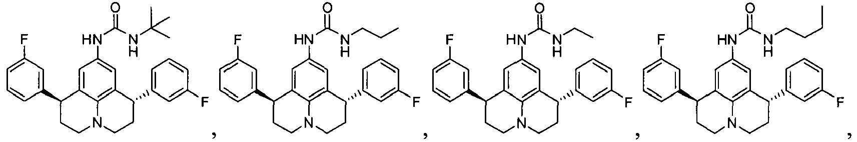

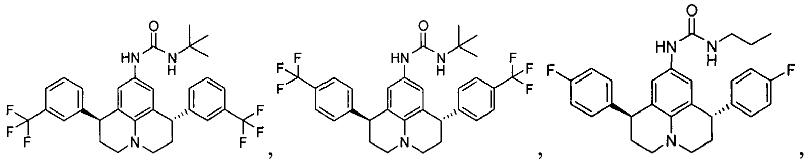

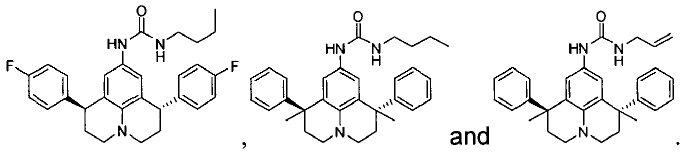

3. A compound or a salt or tautomer thereof according to Claim 1, wherein:
X is O; and
B is C₁₋₆ alkyl or C₁₋₆ haloalkyl.

4. A compound or a salt or tautomer thereof according to Claim 3, which is one of the following compounds or a pharmaceutically-acceptable salt thereof:

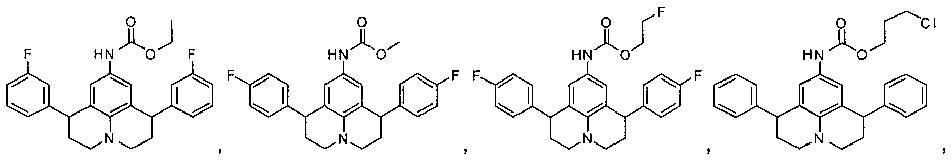

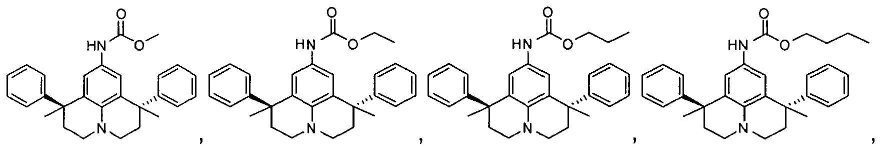

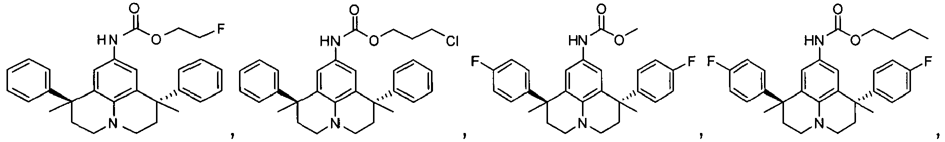

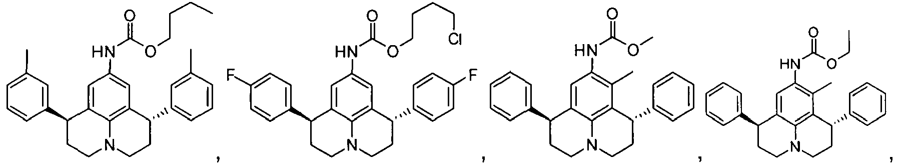

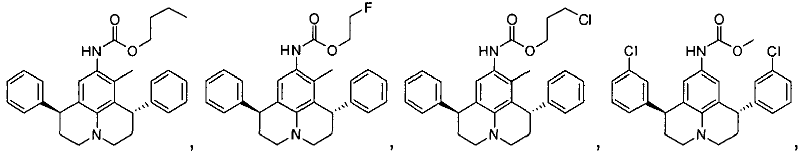

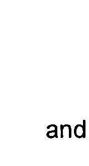

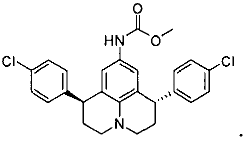

5. A compound or a salt or tautomer thereof according to Claim 1, wherein:
X is S; and
B is C₁₋₆ alkyl;

6. A compound or a salt or tautomer thereof according to Claim 5, which is one of the following compounds or a pharmaceutically-acceptable salt thereof:

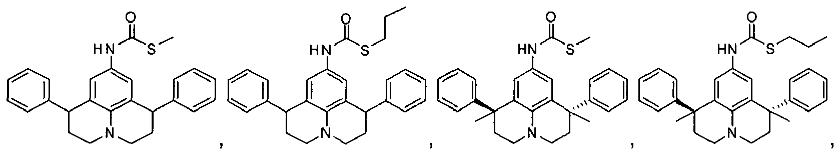

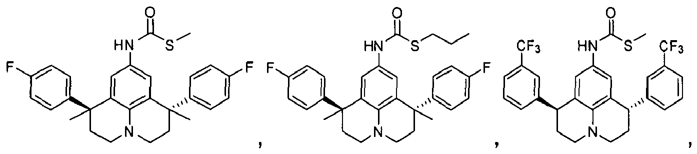

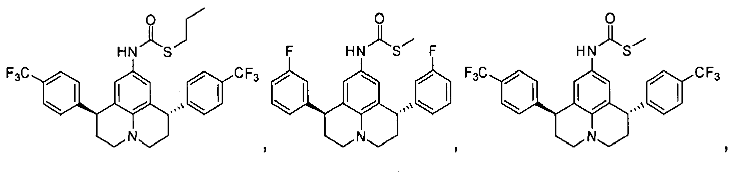

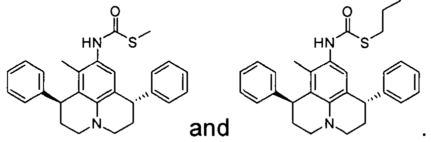

7. A compound or a salt or tautomer thereof according to Claim 1, wherein:
X is NR^{N}; and
B is C₁₋₆ alkyl or C₁₋₆ alkenyl.

8. A compound or a salt or tautomer thereof according to Claim 7, which is one of the following compounds or a pharmaceutically-acceptable salt thereof: 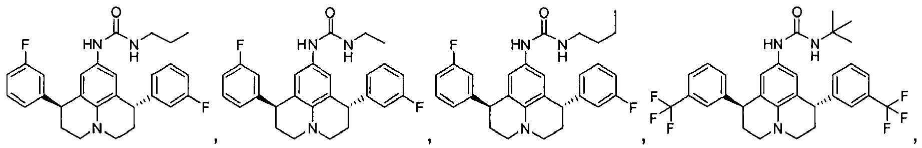 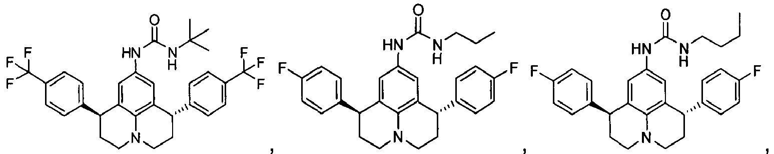 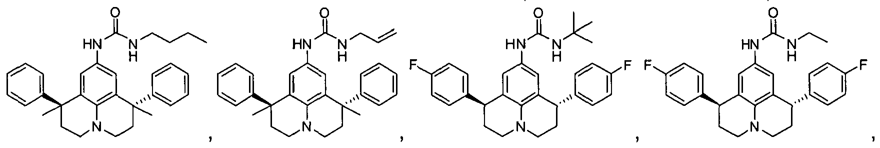 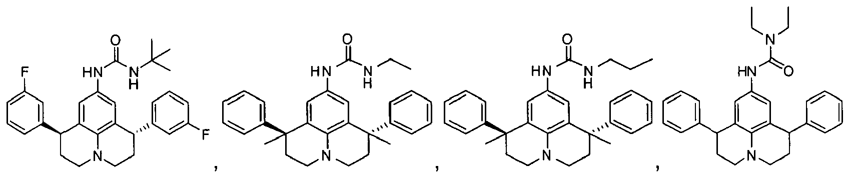 and

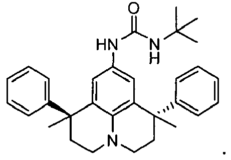

9. A compound selected from the following compounds or a pharmaceutically-acceptable salt or tautomer thereof:

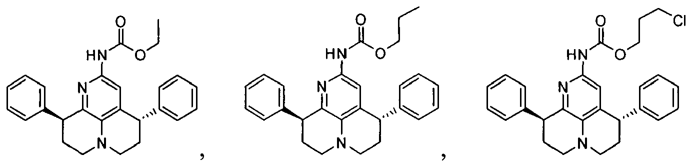

10. A compound or a salt or tautomer thereof according to any preceding claim for use in the treatment of a disease or condition mediated by sphingosine-1-phosphate 3 (S1P3) receptors in a mammal.

11. A compound or a salt or tautomer thereof for use according to Claim 10, wherein the disease or condition is selected from glaucoma, elevated intraocular pressure, ischemic neuropathy, optic neuropathy, visceral pain, corneal pain, headache pain, migraine, cancer pain, back pain, irritable bowel syndrome pain, muscle pain, pain associated with diabetic neuropathy, diabetic retinopathy, a retinal degenerative condition, dry eye, angiogenesis, retinopathy of prematurity, diabetic retinopathy, optic neuropathy, glaucomatous retinopathy, macular degeneration, choroidal neovascularization, an ocular wound, retinal edema, congestive heart failure, cardiac arrhythmia, atherosclerosis, bradycardia, asthma, chronic obstructive pulmonary disease, acute lung injury, acute respiratory distress syndrome, idopathic pulmonary fibrosis, a ventilation-induced lung injury, a skin wound or a cosmetic wound.

12. A compound or a salt or tautomer thereof for use according to Claim 10 or Claim 11, wherein the mammal is a human.

## Patentansprüche

1. Verbindung der nachstehenden Formel oder ein pharmazeutisch annehmbares Salz oder Tautomer davon:: 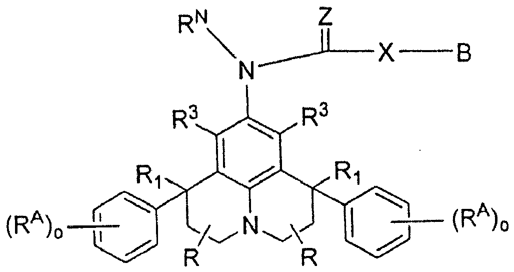 worin:
o 0, 1, 2 oder 3 ist;
X O, S oder NR^{N} ist;
Z O ist;
B C₁₋₆-Alkyl, C₁₋₆-Halogenalkyl oder C₁₋₆-Alkenyl ist;
jedes R, R¹ und R³ unabhängig H oder C₁₋₆-Alkyl ist;
jedes R^{A} unabhängig H, C₁₋₁₂-Alkyl, C₁₋₁₂-Alkenyl, C₁₋₁₂-Alkinyl, Halogen, C₁₋₁₂-Halogenhydrocarbyl, C₁₋₁₂-Hydroxyalkyl, cyclisches C₃₋₁₂-Hydrocarbyl oder Heteroaryl ist; und
R^{N} H oder C₁₋₆-Alkyl ist.

2. Verbindung oder ein Salz oder Tautomer davon gemäss Anspruch 1, die/das eine der nachstehenden Verbindungen oder ein pharmazeutisch annehmbares Salz davon ist:

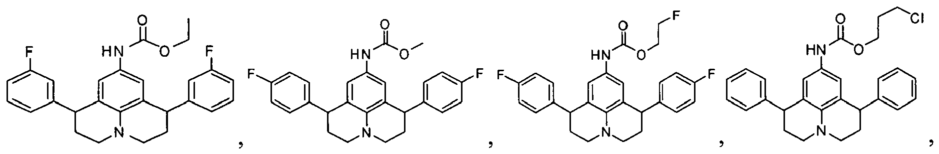

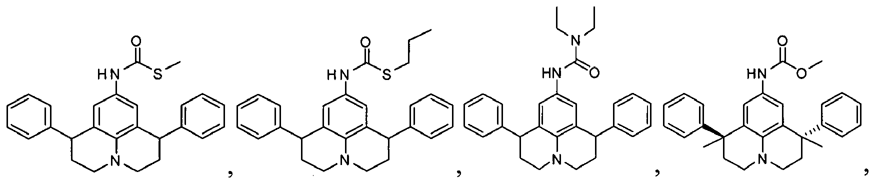

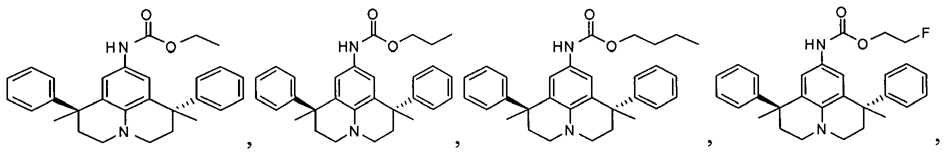

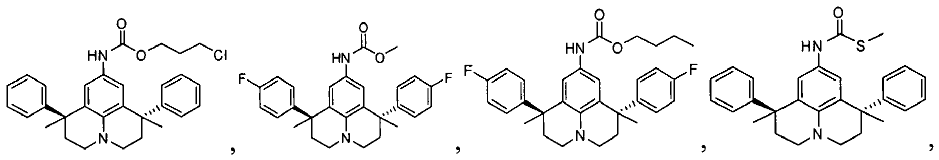

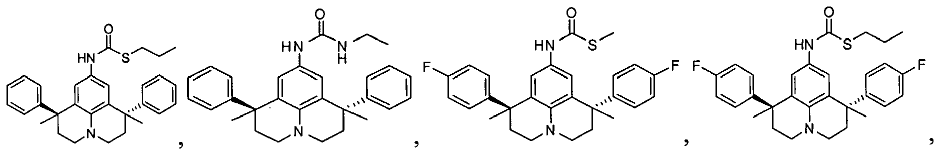

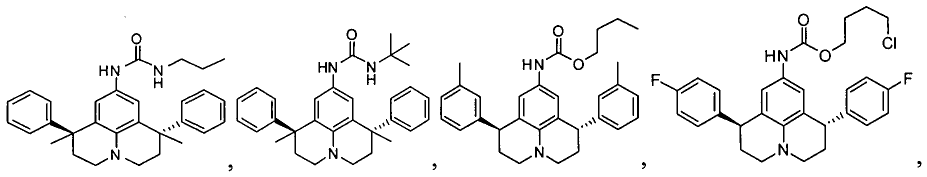

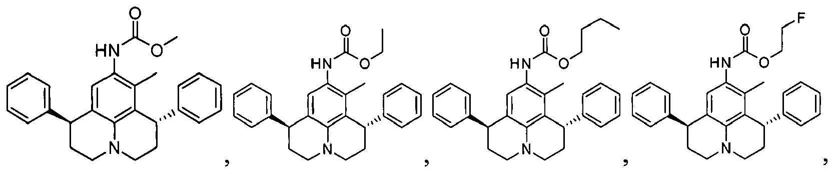

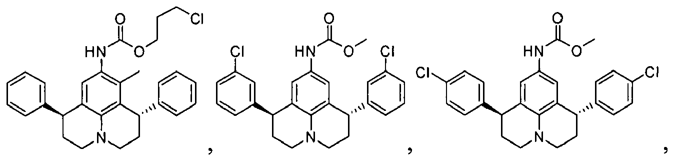

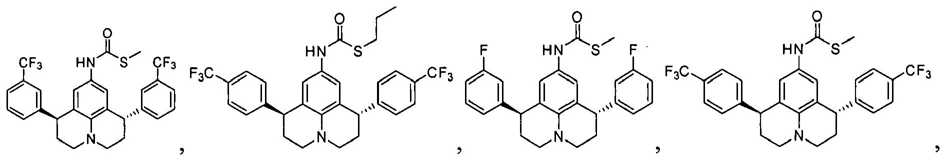

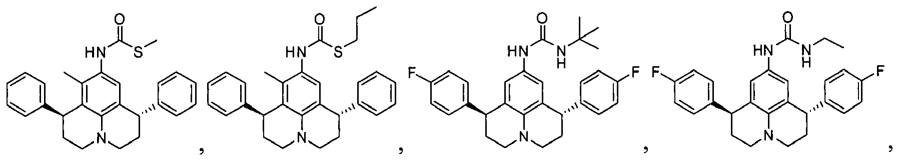

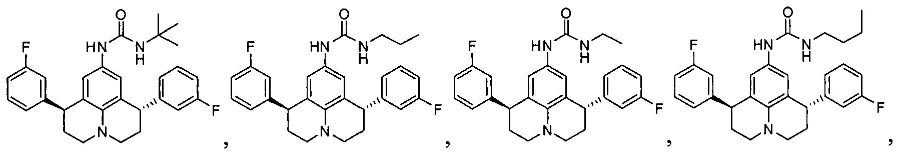

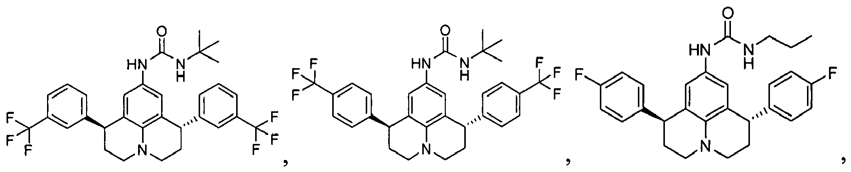

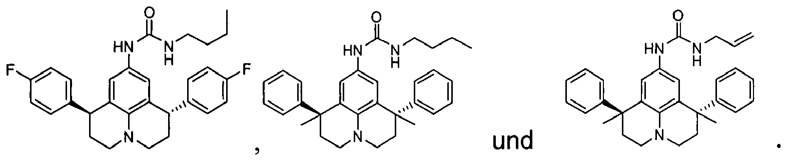

3. Verbindung oder ein Salz oder Tautomer davon gemäss Anspruch 1, worin
X O ist; und
B C₁₋₆-Alkyl oder C₁₋₆-Halogenalkyl ist.

4. Verbindung oder ein Salz oder Tautomer davon gemäss Anspruch 3, die/das eine der nachstehenden Verbindungen oder ein pharmazeutisch annehmbares Salz davon ist: 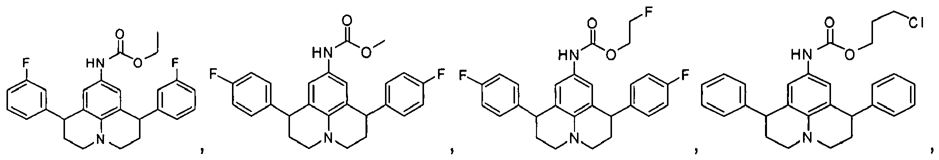 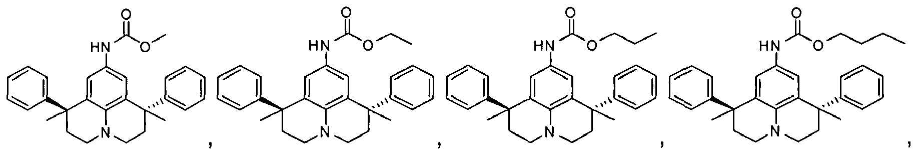 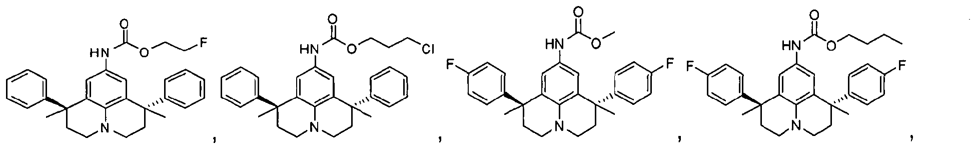 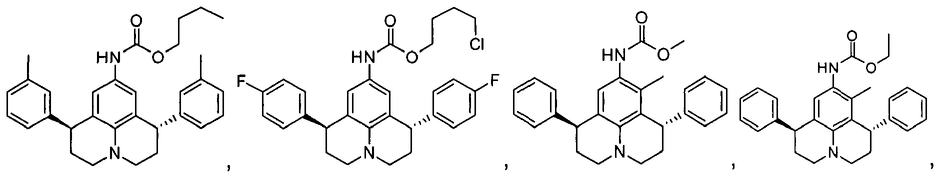 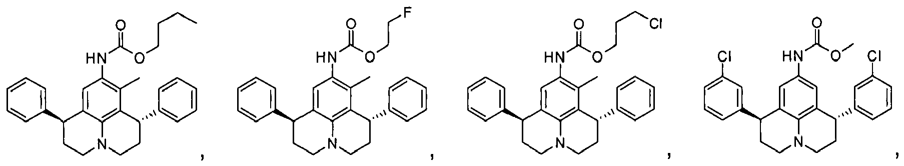 und

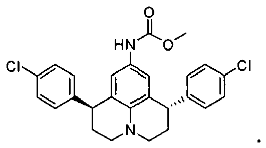

5. Verbindung oder ein Salz oder Tautomer davon gemäss Anspruch 1, worin
X S ist; und
B C₁₋₆-Alkyl ist.

6. Verbindung oder ein Salz oder Tautomer davon gemäss Anspruch 5, die/das eine der nachstehenden Verbindungen oder ein pharmazeutisch annehmbares Salz davon ist:

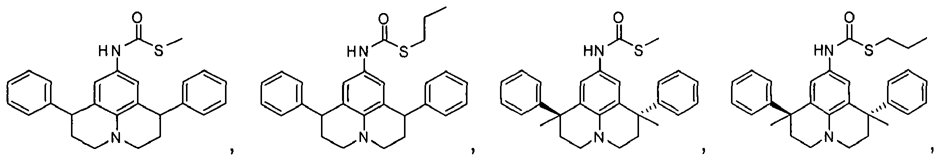

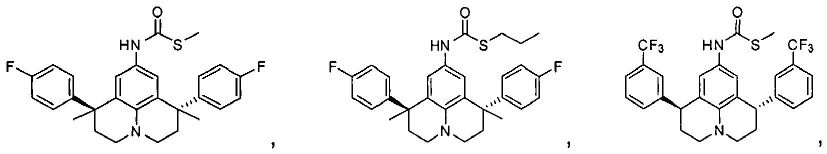

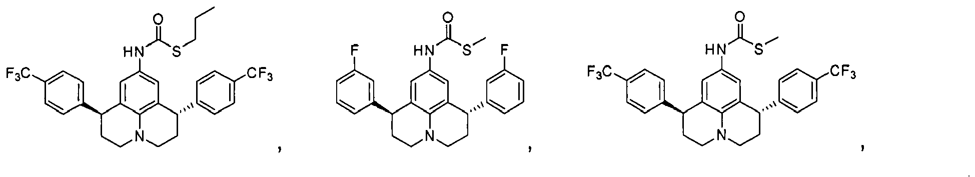

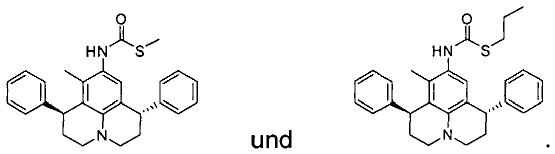

7. Verbindung oder ein Salz oder Tautomer davon gemäss Anspruch 1, worin
X NR^{N} ist; und
B C₁₋₆-Alkyl oder C₁₋₆-Alkenyl ist.

8. Verbindung oder ein Salz oder Tautomer davon gemäss Anspruch 7, die/das eine der nachstehenden Verbindungen oder ein pharmazeutisch annehmbares Salz davon ist: 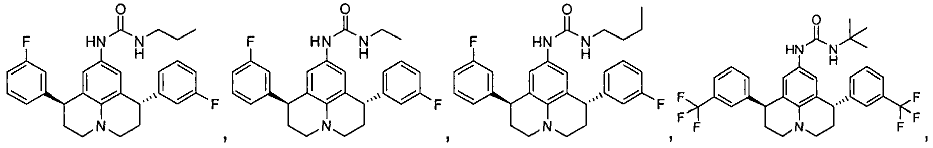 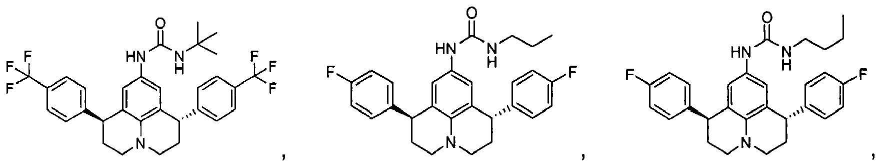 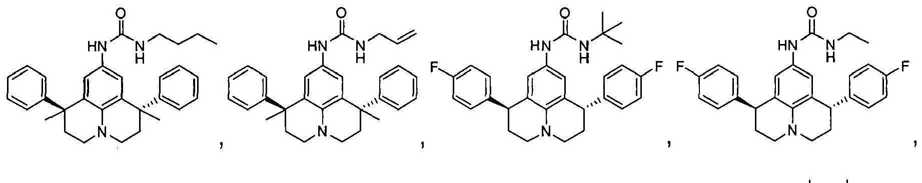 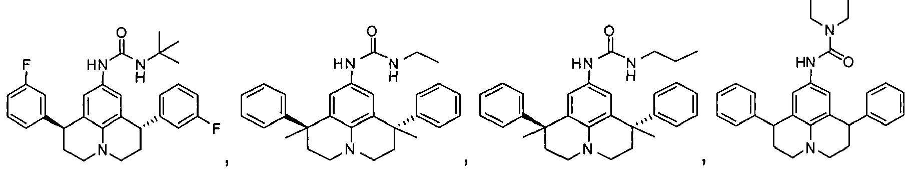 und

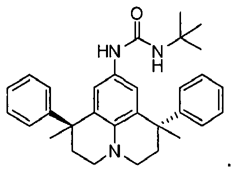

9. Verbindung, ausgewählt aus den nachstehenden Verbindungen, oder ein pharmazeutisch annehmbares Salz oder Tautomer davon:

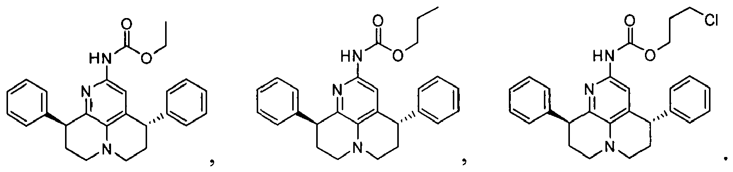

10. Verbindung oder ein Salz oder Tautomer davon gemäss einem der vorhergehenden Ansprüche zur Verwendung bei der Behandlung einer Erkrankung oder eines Zustands, die durch Sphingosin-1-phosphat 3 (S1P3)-Rezeptoren bei einem Säuger vermittelt werden.

11. Verbindung oder ein Salz oder Tautomer davon gemäss Anspruch 10, wobei die Erkrankung oder der Zustand aus Glaukom, erhöhtem Augeninnendruck, ischämischer Neuropathie, optischer Neuropathie, viszeralen Schmerzen, Hornhautschmerzen, Kopfschmerzen, Migräne, Krebsschmerzen, Rückenschmerzen, Reizdarmsyndromschmerzen, Muskelschmerzen, Schmerzen bei diabetischer Neuropathie, diabetischer Retinopathie, degenerativer Netzhauterkrankung, trockenem Auge, Angiogenese, Frühgeborenen-Retinopathie, diabetischer Retinopathie, optischer Neuropathie, glaukomatöser Retinopathie, Makuladegeneration, choroidaler Neovaskularisation, einer Augenwunde, Netzhautödem, Herzinsuffizienz, Herzrhythmusstörungen, Atherosklerose, Bradykardie, Asthma, chronisch obstruktiver Lungenerkrankung, akuter Lungenverletzung, akutem Atemnotsyndrom, idiopathischer Lungenfibrose, einer Belüftungs-induzierten Lungenverletzung, einer Hautwunde oder einer kosmetischen Wunde ausgewählt ist.

12. Verbindung oder ein Salz oder Tautomer davon zur Verwendung gemäss Anspruch 10 oder Anspruch 11, wobei der Säuger ein Mensch ist.

## Revendications

1. Composé représenté par la formule suivante ou un sel ou un tautomère pharmaceutiquement acceptable de celui-ci : 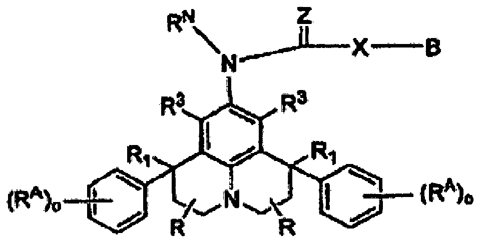 dans laquelle :
o vaut 0 , 1, 2 ou 3 ;
X est 0, S ou NR^{N} ;
Z est O.
B est un alkyle en C₁₋₆, halogénoalkyle en C₁₋₆, ou alcényle en C₁₋₆ ;
chaque R, R¹ et R³ est indépendamment un H ou alkyle en C₁₋₆ ;
chaque R^{A} est indépendamment un H, alkyle en C₁₋₁₂, alcényle en C₁₋₁₂, alcynyle en C₁₋₁₂, halogéno, halogénohydrocarbyle en C₁₋₁₂, hydroxyalkyle en C₁₋₁₂, hydrocarbyle cyclique en C₃₋₁₂ ou hétéroaryle ; et
R^{N} est un H ou alkyle en C₁₋₆.

2. Composé ou un sel ou un tautomère de celui-ci selon la revendication 1, qui est l'un des composés suivants ou un sel pharmaceutiquement acceptable de celui-ci :

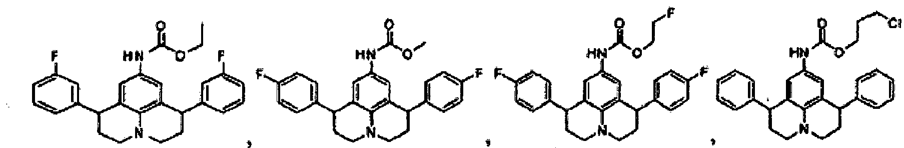

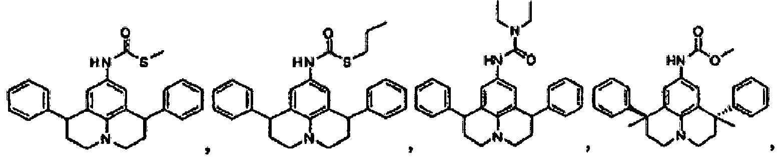

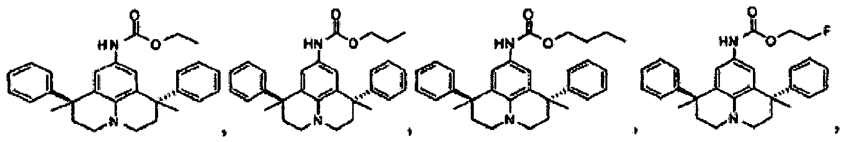

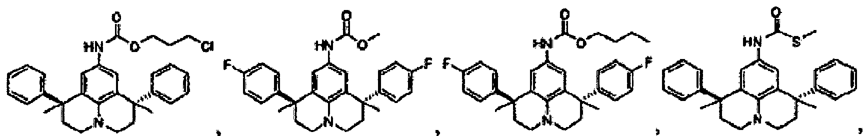

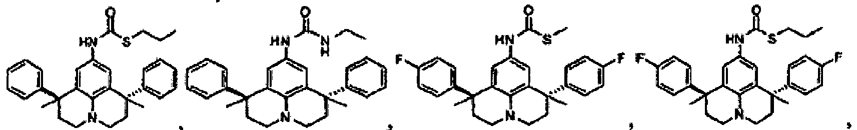

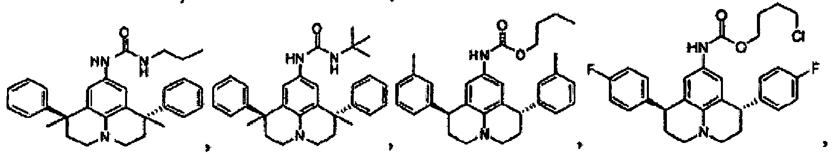

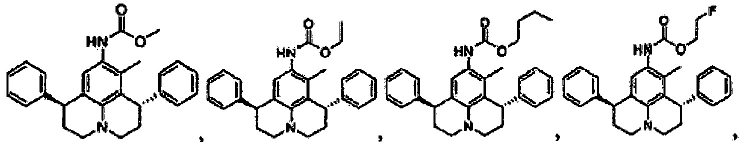

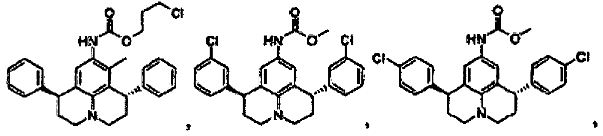

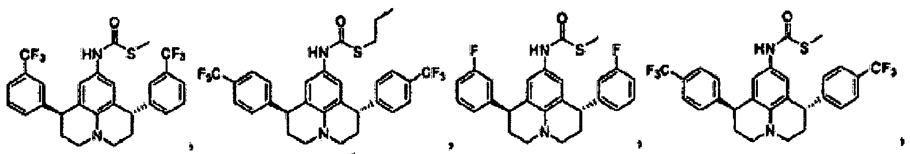

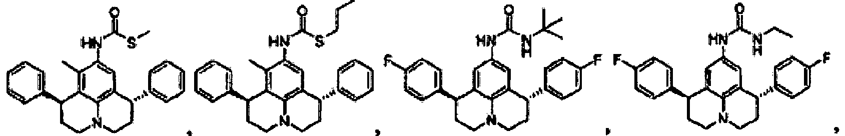

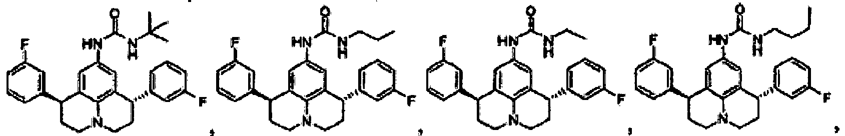

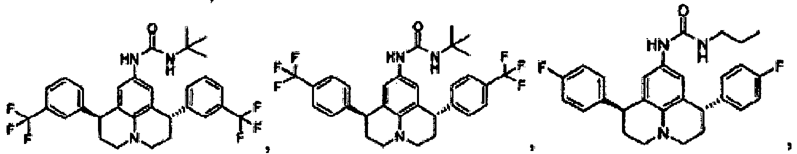

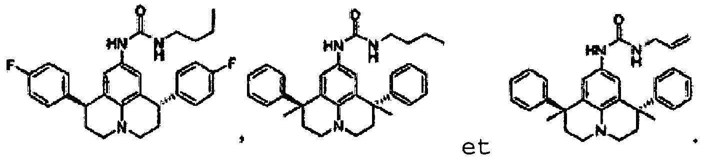

3. Composé ou un sel ou un tautomère de celui-ci selon la revendication 1, dans lequel:
X est O ; et
B est un alkyle en C₁₋₆ ou halogénoalkyle en C₁₋₆.

4. Composé ou un sel ou un tautomère de celui-ci selon la revendication 3, qui est l'un des composés suivants ou un sel pharmaceutiquement acceptable de celui-ci : 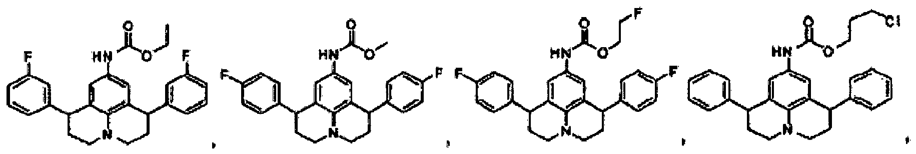 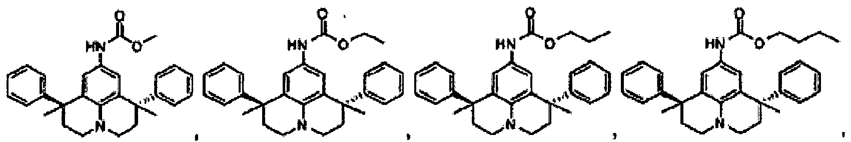 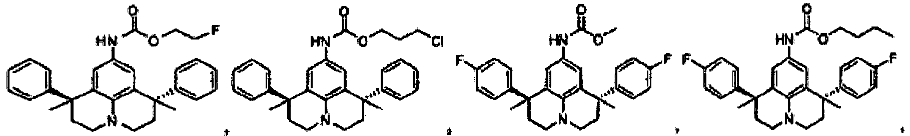 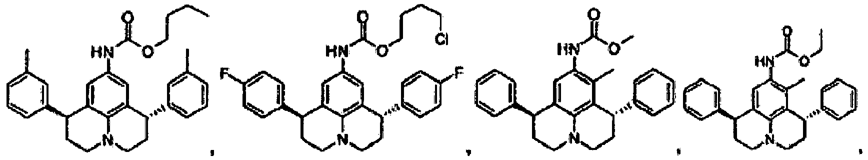 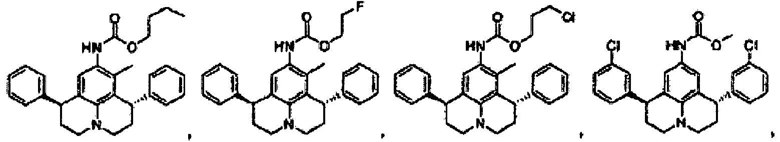 et

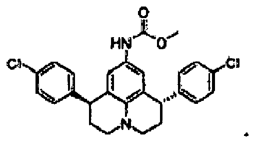

5. Composé ou un sel ou un tautomère de celui-ci selon la revendication 1, dans lequel :
X est S ; et
B est un alkyle en C₁₋₆.

6. Composé ou un sel ou un tautomère de celui-ci selon la revendication 5, qui est l'un des composés suivants ou un sel pharmaceutiquement acceptable de celui-ci :

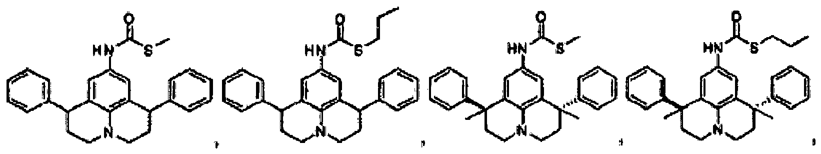

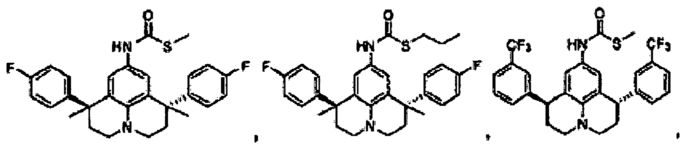

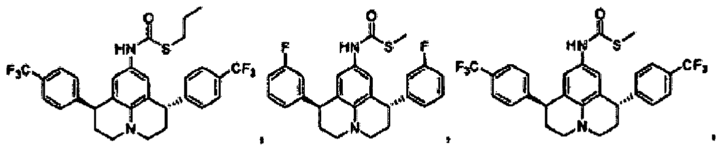

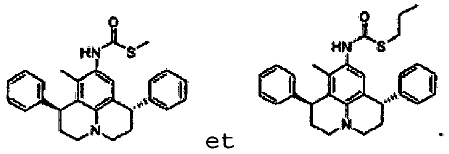

7. Composé ou un sel ou un tautomère de celui-ci selon la revendication 1, dans lequel :
X est NR^{N} ; et
B est un alkyle en C₁₋₆ ou alcényle en C₁₋₆.

8. Composé ou un sel ou un tautomère de celui-ci selon la revendication 7, qui est l'un des composés suivants ou un sel pharmaceutiquement acceptable de celui-ci : 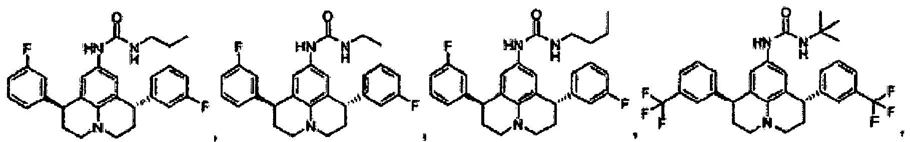 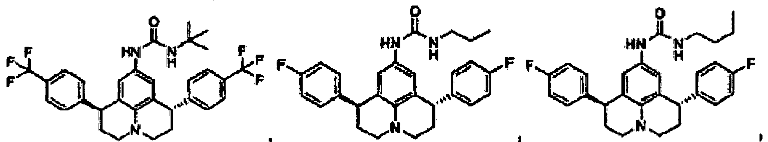 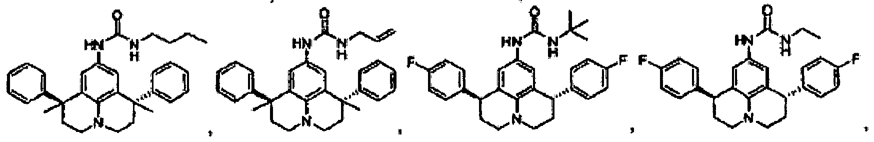 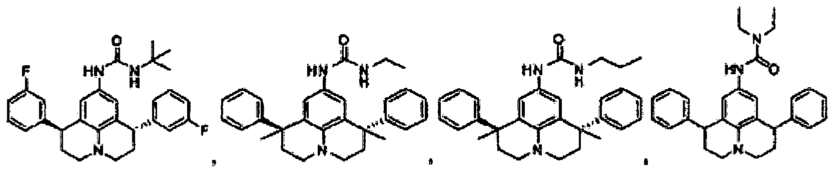 et

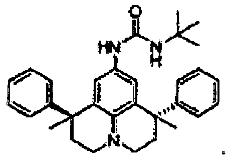

9. Composé choisi parmi les composés suivants ou un sel ou un tautomère pharmaceutiquement acceptable de celui-ci :

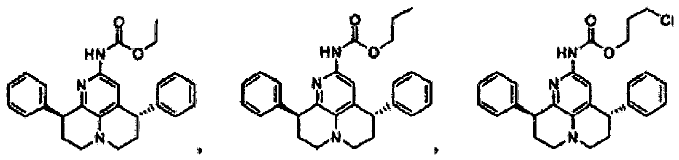

10. Composé ou un sel ou un tautomère de celui-ci selon l'une quelconque des revendications précédentes destiné à être utilisé dans le traitement d'une maladie ou d'une affection médiée par les récepteurs de la sphingosine-1-phosphate 3 (S1P3) chez un mammifère.

11. Composé ou un sel ou un tautomère de celui-ci destiné à être utilisé selon la revendication 10, dans lequel la maladie ou l'affection est choisie parmi un glaucome, une pression intraoculaire élevée, une neuropathie ischémique, une neuropathie optique, une douleur viscérale, une douleur de la cornée, une douleur de type céphalée, une migraine, une douleur cancéreuse, une dorsalgie, une douleur due au syndrome du colon irritable, une douleur musculaire, une douleur associée à une neuropathie diabétique, une rétinopathie diabétique, une affection dégénérative de la rétine, une sécheresse oculaire, l'angiogenèse, la rétinopathie de la prématurité, une rétinopathie diabétique, une neuropathie optique, une rétinopathie glaucomateuse, une dégénérescence maculaire, une néovascularisation choroïdienne, une lésion oculaire, un oedème de la rétine, une défaillance cardiaque congestive, une arythmie cardiaque, une athérosclérose, une bradycardie, un asthme, une bronchopneumopathie obstructive chronique, une lésion pulmonaire aigüe, un syndrome de détresse respiratoire aigu, une fibrose pulmonaire idiopathique, une lésion pulmonaire induite par la ventilation, une blessure cutanée ou une blessure par produit cosmétique.

12. Composé ou un sel ou un tautomère de celui-ci destiné à être utilisé selon la revendication 10 ou la revendication 11, dans lequel le mammifère est un humain.
